# EUROPÄISCHE PATENTSCHRIFT

(11) **EP 0 341 681 B1**
(45) Veröffentlichungstag und Bekanntmachung des Hinweises auf die Patenterteilung: **29.12.1993**
(21) Anmeldenummer: 89108378.4
(22) Anmeldetag: 10.05.1989
(51) Int. Cl.: C07D 309/30, C07C 39/367, C07C 59/64, C07C 323/01, A61K 31/35, A61K 31/185

(54) **Neue 6-Phenoxymethyl-4-hydroxytetrahydropyran-2-one und 6-Thiophenoxymethyl-4-hydroxytetrahydropyran-2-one, sowie die entsprechenden Dihydroxycarbonsäurederivate, Salze und Ester, Verfahren zur Herstellung dieser Verbindungen, ihre Verwendung als Arzneimittel, pharmazeutische Präparate und neue Phenole sowie Thiophenole**
6-Phenoxymethyl-4-hydroxytetrahydropyran-2-ones and 6-thiophenoxymethyl-4-hydroxytetrahydropyran-2-ones, the corresponding dihydrocarboxylic-acid derivatives, salts and esters, process for preparing these compounds, their use as medicines, pharmaceutical compositions and phenols and thiophenols
6-Phénoxyméthyl-4-hydroxytétrahydropyrane-2-ones et 6-thiophénoxyméthyl-4-hydroxytétrahydropyrane-2-ones et les dérivés d'acide dihydroxycarboxyliques correspondants, sels et esters, procédé pour préparer ces composés, leur usage comme médicaments, compositions pharmaceutiques et phénols et thiophénols

(30) Priorität: 13.05.1988 DE 3816388; 11.06.1988 DE 3819999
(43) Veröffentlichungstag der Anmeldung: 15.11.1989
(73) Patentinhaber: HOECHST AKTIENGESELLSCHAFT, 65926 Frankfurt am Main (DE)
(72) Erfinder: Jendralla, Heiner, Dr., D-6230 Frankfurt am Main 80 (DE); Wess, Günther, Dr., D-6455 Erlensee (DE); Bartmann, Wilhelm, Prof. Dr., D-6232 Bad Soden am Taunus (DE); Beck, Gerhard, Dr., D-6000 Frankfurt am Main (DE)

(56) Entgegenhaltungen:
- EP-A- 0 216 127
- DE-A- 3 632 893

## Beschreibung

Das Enzym 3-Hydroxy-3-methylglutaryl-Coenzym-A-Reduktase (HMG-CoA-Reduktase) spielt eine zentrale Rolle bei der Biosynthese des Cholesterins [A. Endo, J. Med. Chem. 28, 401 (1985)]. Hemmstoffe dieses Enzyms, insbesondere Mevinolin [A.S. Pappu et al., Clin. Res. 34, 684 A (1986)], Synvinolin [A.S. Olsson et al., The Lancet, 391 (1986); M.J.T.M. Mol et al., The Lancet, 936 (1986)] und Eptastatin [Drugs of the Future 12, 437 (1987); N. Nakaya et al. Atherosclerosis 61, 125 (1986)] wurden zur Behandlung von Hypercholesterolämikern klinisch getestet. Strukturell vereinfachte, vollsynthetische Analoga dieser Verbindungen wurden beschrieben [G.E. Stokker et al., J. Med. Chem. 29, 170 und 852 (1986), W.F. Hoffman et al., J. Med. Chem. 29, 159 (1986)].

In der Europäischen Patentanmeldung A-0216127 (entsprechend US Patentanmeldung Nr. 900 848) werden Verbindungen der Formel Ia 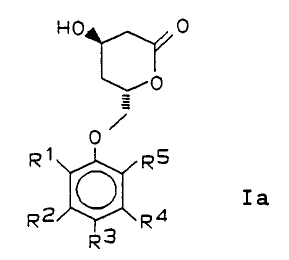
worin
R¹ und R⁵ gleich oder verschieden sind und a) Wasserstoff oder Halogen, b) Cycloalkyl mit 4-8 C-Atomen oder einen Phenylrest, der im Kern 1- bis 3-fach substituiert sein kann mit Halogen, Trifluormethyl und/oder Alkyl oder Alkoxy mit je 1-4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1- bis 3-fach substituiert sein können mit
α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy oder Alkinyloxyresten mit 3 bis 6 Kohlenstoffatomen,
β) Halogen, Hydroxy, Cycloalkyl mit 3-7 C-Atomen, unsubstituierten Phenyl-, α- oder β-Thienylresten, oder Phenyl-, α- oder β-Thienylresten, welche ihrerseits im Kern 1- bis 3-fach substituiert sind mit Halogen, Trifluormethyl und/oder Alkyl oder Alkoxy mit 1-4 C-Atomen,
γ) unsubstituierten Phenoxy-, Benzyloxy-, α- oder β-Thienyloxyresten, oder Phenoxy-, Benzyloxy-, α- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,
δ) der Gruppe 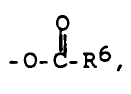 wobei R⁶ bedeutet:
   einen geradkettigen oder verzweigten Alkyl- oder Alkenylrest mit bis zu 8 Kohlenstoffatomen, oder einen Cycloalkyl oder Cycloalkenylrest mit jeweils 3-8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1-4 C-Atomen, oder einen 3-Pyridylrest,
R² und R⁴, gleich oder verschieden sind und Wasserstoff, Alkyl mit 1-4 C-Atomen, Halogen oder Alkoxy mit 1-4 C-Atomen bedeuten, und
R³ Wasserstoff, Alkyl oder Alkenyl mit bis zu 4 C-Atomen, Halogen oder Alkoxy mit 1-4 C-Atomen ist,
sowie die entsprechenden offenkettigen Dihydroxycarbonsäuren, deren pharmakologisch verträgliche Salze mit Basen und deren pharmakologisch verträgliche Ester, beansprucht.

Die in dieser Anmeldung beschriebenen Verbindungen hemmen die HMG-CoA-Reduktase mit IC₅₀-Werten im 10⁻⁵ bis 10⁻⁸ molaren Bereich. Entsprechenden Angaben in der Beschreibung hat die wirkstärkste Verbindung Ia (R¹ = R³ = Cl, R² = R⁴ = H, 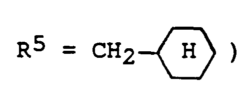
IC₅₀ = 2,5 x 10⁻⁸ M.

Die deutsche Offenlegungsschrift 36 32 893 (= Derwent Referat 88-99 366/15) betrifft u.a. Verbindungen der allgemeinen Formel Ib 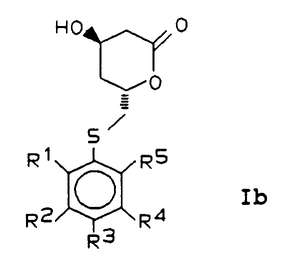
worin
- R¹ und R⁵: gleich oder verschieden sind und
a) Wasserstoff oder Halogen
b) einen Phenylrest, der im Kern 1- bis 3-fach substituiert sein kann mit Halogen, Trifluormethyl, Methyl, Ethyl, Methoxy, Ethoxy
c) einen Alkylrest mit 1-5 Kohlenstoffatomen, der 1- bis 3-fach substituiert sein kann mit
   α) C₁-C₃-Alkoxyresten oder Cycloalkoxyresten mit 3 bis 7 Kohlenstoffatomen
   β) Phenoxy- oder Benzyloxyresten, welche ihrerseits im Kern 1- bis 3-fach substituiert sein können mit Halogen, Trifluormethyl, Methyl, Ethyl, Methoxy, Ethoxy,
   γ) Halogen, Cycloalkyl mit 3-7 C-Atomen, Phenylresten, welche ihrerseits im Kern 1- bis 3-fach substituiert sein können mit Halogen, Trifluormethyl, Methyl, Ethyl, Isopropyl, Methoxy, Ethoxy,
   δ) 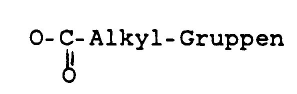 mit insgesamt 3-8 Kohlenstoffatomen, bedeuten,
- R² und R⁴: gleich oder verschieden sind und Wasserstoff, Halogen, Methyl, Ethyl, Methoxy, Ethoxy, Benzyloxy bedeuten,
- R³: Wasserstoff, Halogen, Trifluormethyl, Methyl, Ethyl, Methoxy, Ethoxy ist,
sowie die entsprechenden offenkettigen Dihydroxycarbonsäuren, deren pharmakologisch verträgliche Salze mit Basen und deren pharmakologisch verträgliche Ester.

Die beschriebenen Verbindungen der Formel Ib sind entsprechend den Angaben in dieser Anmeldung in der Regel bei gleichem Substitutionsmuster R¹-R⁵ geringfügig schwächer wirksam als Ia.

Es wurden nun überraschenderweise in diesen Anmeldungen nicht durch Beispiele beschriebene Substitutionsmuster (R¹ bis R⁵) aufgefunden, die Verbindungen der allgemeinen Formeln Ia und Ib eine bis zu einer Zehnerpotenz höhere Wirksamkeit als den besten in EP-A-0216127 und DE-A-36 32 893 aufgeführten Beispielen verleihen. Die Substitutionsmuster liegen bezüglich der Substituenten R¹, R², R⁴, R⁵ vollständig innerhalb, bezüglich R³ nur teilweise innerhalb der allgemeinen Patentansprüche von EP-A-0216127 und DE-A-36 32 893, denn es wurde ferner gefunden, daß R³ auch Bedeutungen aufweisen kann, die in den beiden älteren Anmeldungen nicht aufgeführt sind.

Die Erfindung betrifft neue Verbindungen der allgemeinen Formel I 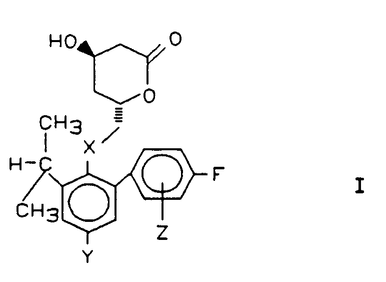
sowie die entsprechenden offenkettigen Dihydroxycarbonsäuren der Formel II, 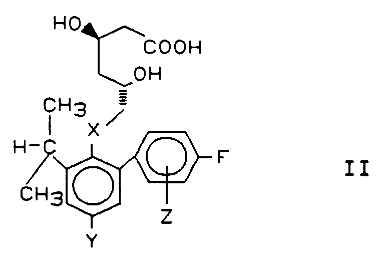
deren pharmakologisch verträgliche Salze mit Basen und deren pharmakologisch verträgliche Ester.
In den Formeln bedeuten
- X: Sauerstoff oder Schwefel,
- Y: a) einen geradkettigen oder verzweigten Alkylrest mit 3 bis 12 Kohlenstoffatomen
b) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 C-Atomen und
- Z: Wasserstoff oder einen geradkettigen oder verzweigten Alkylrest mit 1 bis 4 Kohlenstoffatomen.

Für Verbindungen der Formeln I und II, welche nicht unter die Patentansprüche der genannten älteren Patentanmeldungen fallen, wird der Charakter einer Auswahlerfindung geltend gemacht.

Bevorzugt bedeuten die Substituenten in den allgemeinen Formeln:
- X: Sauerstoff
- Y: Isopropyl, tert.-Butyl, Cyclohexyl, Phenyl, p-Fluorphenyl
- Z: Wasserstoff.

Die Erfindung betrifft ferner ein Verfahren zur Herstellung der Verbindungen der Formel I sowie der entsprechenden offenkettigen Dihydroxycarbonsäuren der Formel II, von deren pharmakologisch verträglichen Salzen mit Basen und deren pharmakologisch verträglichen Estern. Das Verfahren ist dadurch gekennzeichnet, daß man
a) entsprechend substituierte Phenole oder Thiophenole der Formel III 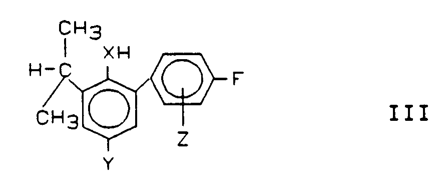 worin X, Y und Z die zu den Formeln I und II angegebenen Bedeutungen haben, mit dem optisch reinen Jodid der Formel IV 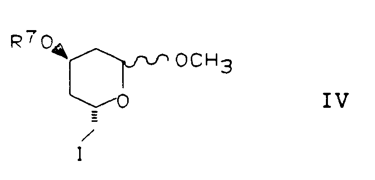 worin R⁷ eine gegen Basen und schwache Säuren stabile Schutzgruppe bedeutet, in die Lactolether der Formel V 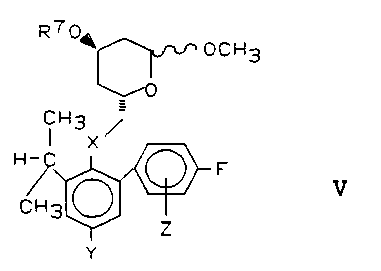 worin X, Y und Z die zu den Formeln I und II und R⁷ die zu Formel IV angegebenen Bedeutungen haben, überführt,
b) die Lactolether der Formel V zu den entsprechenden Lactolen der Formel VI 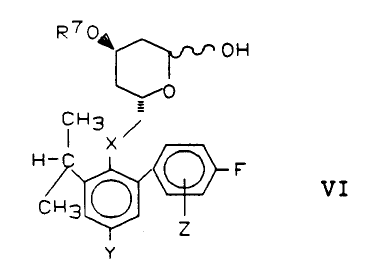 worin X, Y und Z die zu den Formeln I, II und R⁷ die zu Formel IV angegebene Bedeutungen haben, hydrolysiert,
c) die Lactole der Formel VI zu den entsprechenden Lactonen der Formel VII 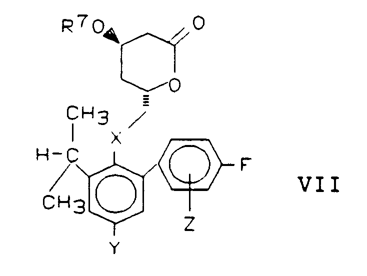 worin X, Y und Z die zu den Formeln I/II und R⁷ die zu Formel IV angegebenen Bedeutungen haben, oxydiert,
d) die erhaltenen geschützten Lactone der Formel VII in an sich bekannter Weise in Verbindungen der Formel I überführt und
e) gegebenenfalls die erhaltenen Verbindungen der Formel I in die entsprechenden offenkettigen Dihydroxycarbonsäuren der Formel II, deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

Das Verfahren wird zweckmäßigerweise unter den Bedingungen durchgeführt, die in den genannten älteren Anmeldungen beschrieben wurden. Je nach Bedeutung der Substituenten können die Verfahrensbedingungen modifiziert werden (vgl. z.B. Ausführungsbeispiel 1.8)
Die Ausgangsverbindungen der Formel III sind neu. Die Erfindung betrifft daher auch diese Verbindungen. Die Jodide der Formel IV sind z.B. in EP-A-0216127 beschrieben.

Synthesen der benötigten Phenol- bzw. Thiophenolbausteine III sind in dem Formelschema 1 skizziert und nachfolgend erläutert.
Verbindungen der Formel III können aus 2-Isopropylphenol XII bzw. aus in 4-Stellung mit Y substituierten 2-Isopropylphenolen XIII durch Palladium (O) katalysierte Aryl-Aryl-Kupplung als Schlüsselschritt erhalten werden. Übersichten über Palladium (O) katalysierte Aryl-Aryl-Kupplung findet man bei E. Negishi, Acc. Chem. Res. 15, 340 (1982) und R.F. Heck "Palladium Reagents in Organic Synthesis", Academic Press (1985), Kap. 6.

Nach einer jüngst publizierten Strategie (D.A. Widdowson, Y.-Z. Zhang, Tetrahedron 42, 2111 (1986)) weisen Arylgrignard-Verbindungen eine erhöhte Reaktivität bezüglich Pd(O)-katalysierter Kupplung mit Arylhalogeniden auf, wenn sie ortho-Alkoxysubstituenten tragen. Bromiert man deshalb XIII zu XIV, schützt dann XIV mit einer Benzylgruppe unter Bildung von XV und formt das Grignard-Reagenz XVI in THF, so reagiert dieses bereits unter milden Bedingungen (10 bis 65°C) in hervorragenden Ausbeuten (90 bis 98 %) unter Pd(O)-Katalyse mit dem Arylhalogenid XVII (Hal = Br oder I) zum Kupplungsprodukt XVIII. Die Entfernung der Schutzgruppe mittels katalytischer Hydrierung liefert III (X = O). Bei dieser Strategie erhält man das Kupplungsprodukt III (X = O) in sehr hoher Ausbeute und Reinheit. Bei diesem Verfahren besteht die Notwendigkeit die phenolische OH-Gruppe zu schützen und nachträglich die Schutzgruppe zu entfernen.

Pd(O)-katalysierte Aryl-Vinyl-Kupplungen unter Anwesenheit ungeschützter Phenolgruppen sind bekannt (R.F. Heck, Acc. Chem. Res. 12, 146 (1979); C.B. Ziegler Jr., R.F. Heck, J. Org. Chem. 43, 2941 (1978)). Diese Reaktion ist mit Aryl-Aryl-Kupplungen nicht voll vergleichbar, da dort keine hochbasischen metallorganischen Reagenzien (wie die Grignard-Verbindung XX) zum Einsatz kommen.

Aryl-Aryl-Kupplungen in Anwesenheit ungeschützter Phenolgruppen sind neu. Wir haben erfolgreich diese Umkehrung der oben beschriebenen konventionellen Strategie durchgeführt, nämlich die Pd(O)-katalysierte Umsetzung von ungeschützten ortho-Jodphenolen XIX mit Grignard-Reagenzien aus p-Bromfluorbenzolen XX. Ein Äquivalent von XX wird zur Deprotonierung von XIX und ein weiteres Äquivalent XX wird zur Kupplungsreaktion verbraucht. Da es darüber hinaus als Nebenreaktion zur Oligomerisierung der Grignard-Komponente XX kommt, muß man zur Erzielung eines vollständigen Umsatzes zu III (X = O) 2,5 bis 3,0 Äquivalente XX einsetzen.

Es gelingt auf diese Weise auch quantitative Dikupplungen an ungeschütztem Dijodid XXI vorzunehmen. Arbeitet man mit 3 Äquivalenten XX, so erhält man bei Raumtemperatur ein Gemisch des Monokupplungsprodukts XIX′ 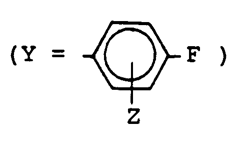
und des Dikupplungsprodukts III′ 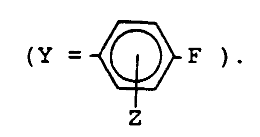
Arbeitet man hingegen mit ≧ 4 Äquivalenten XX, so setzt sich das zwischenzeitlich nachweisbare Monokupplungsprodukt letztlich vollständig zu III′ um. Da diese Dikupplungsreaktion ohne Reinigung des recht empfindlichen * Dijodids XXI gelingt, erhält man III′ 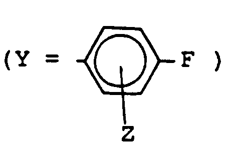
sehr direkt aus XII mit 40 bis 60 % Gesamtausbeute (nicht optimiert!).
* siehe C.V. Bordlianu, Arch. d. Pharmazie 272, 8 (1934)

Dieser Phenolbaustein III′ wird zweckmäßigerweise nach diesem Verfahren hergestellt, da man sich bei der konventionellen Methode der ohnehin entstehenden Verbindung XIX′ bedienen müßte, da Aryl-digrignard-Verbindungen instabil sind [F. Bickelhaupt, Angew. Chem. 99, 1020 (1987)].

Die direkte Kupplung ungeschützter Jodphenole XIX mit Arylgrignard-Verbindungen spart verglichen mit der konventionellen Kupplung zwei Syntheseschritte und erlaubt den Einsatz der billigeren Fluorbrombenzole. Dafür muß eine niedrigere Ausbeute des Kupplungsschritts in Kauf genommen werden.

Als Palladium-Katalysatoren haben wir Tetrakis(triphenylphosphin)palladium (O), Bis(triphenylphosphin)palladiumdichlorid oder ein Gemisch aus Palladiumdichlorid und Triphenylphosphin eingesetzt. Es ist bekannt, daß ähnliche Katalysen auch mit Nickelphosphin-Komplexen und verwandten Übergangsmetall-Komplexen erzielt werden können [s. z.B. E. Negishi, Acc. Chem. Res. 15, 340 (1982); J.K. Stille, Angew. Chem. 98, 504 (1986); R.F. Heck, Acc. Chem. Res. 12, 146 (1979); E. Negishi et al., J. Org. Chem. 42, 1821 (1977)].

Die Thiophenole der Formel III erhält man analog den in der Literatur beschriebenen Methoden aus den entsprechenden Phenolen der Formel III durch Reaktion mit einem Dialkylthiocarbamidsäurechlorid, anschließende thermische Newman-Kwart-Umlagerung und reduktive Spaltung des entstehenden S-Aryldialkylthiocarbamats zu Thiophenolen der Formel III (vgl. auch DE-A-36 32 893).

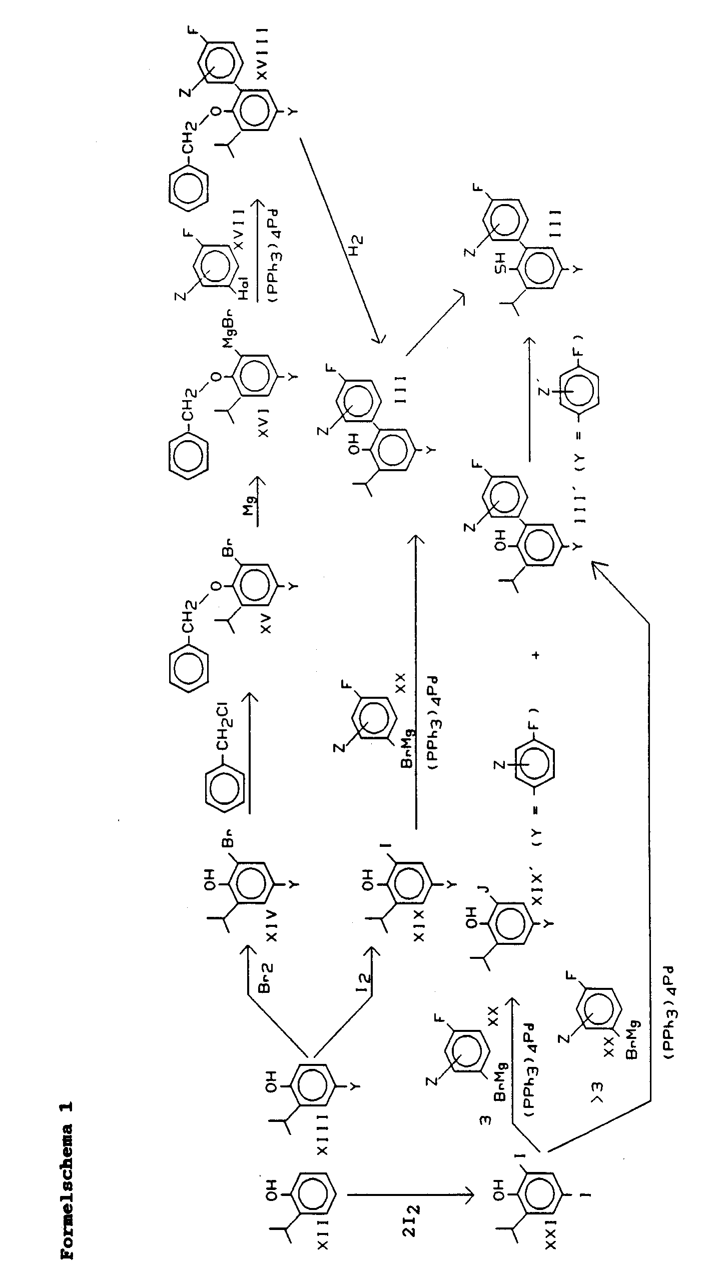

Synthesewege zur Herstellung von Verbindungen der Formel XIII (Formelschema 1) sind z.T. im Formelschema 2 skizziert und nachstehend beschrieben.

Die Herstellung der Ausgangsverbindung XIII mit bestimmten Substituenten Y richtet sich nach der Verfügbarkeit von Ausgangsmaterialien.

XIII (Y = i-Pr) entsteht durch Decarboxylierung von kommerziell erhältlicher 3,5-Diisopropyl-2-hydroxybenzoesäure der Formel XI (Janssen). Man kann die Decarboxylierung durch Erhitzen der Reinsubstanz oder der Lösung in einem inerten Lösungsmittel (z.B. Nitrobenzol) auf 210 bis 220°C durchführen. Wesentlich bessere Ausbeute und Reinheit erhält man, wenn man die Lösung in Chinolin in Anwesenheit eines Kupferchromit-Katalysators auf ca. 190°C erhitzt.

XIII (Y = tert.-Butyl) erhält man hoch para-selektiv nach G. Sartori et al., Chem. and Industry, 762 (1985), wenn man Isopropylphenol in CH₂Cl₂-Lösung mit Methyl-tert.-butylether (MTBE) und Zirkonium(IV)chlorid rührt. Gute Ausbeute an XIII erhält man, wenn man XII (1.0 Aquiv.) mit MTBE (1.05 Äquiv.) und ZrCl₄ (2.4 Äquiv. in 2 Portionen) bei 0°C umsetzt.

Auch konventionelle Friedel-Crafts-Alkylierungen von XII mit entsprechenden Alkylhalogeniden/Aluminiumtrichlorid oder mit Alkoholen Y-OH/Lewis- oder Protonsäuren können zur Herstellung von XIII dienen [siehe K.-D. Bode in Houben-Weyl "Methoden der organischen Chemie" Bd. VI/1c "Phenole Teil 2", Georg Thieme Verlag, Stuttgart (1976), Seite 925 ff.].

4-Hydroxy-3-isopropyl-biphenyl XIII 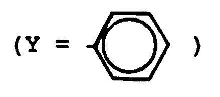
erhält man aus käuflichem p-Nitrobiphenyl VIII durch o-Alkylierung mit Isopropylmagnesiumbromid [Übersicht G. Bartoli, Acc. Chem. Res. 17, 109 (1984)] zu IX, Reduktion von IX zum Amin X, Diazotierung und Phenolverkochung (Formelschema 2).

Die katalytische Hydrierung einer Ethylacetat-Lösung von XIII 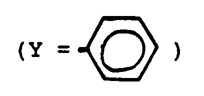
über 5 % Palladium auf Kohle bei 50°C und 4 bis 6 kg cm⁻² Wasserstoffdruck ergibt in 85 bis 90 %iger Ausbeute XIII

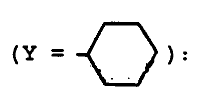

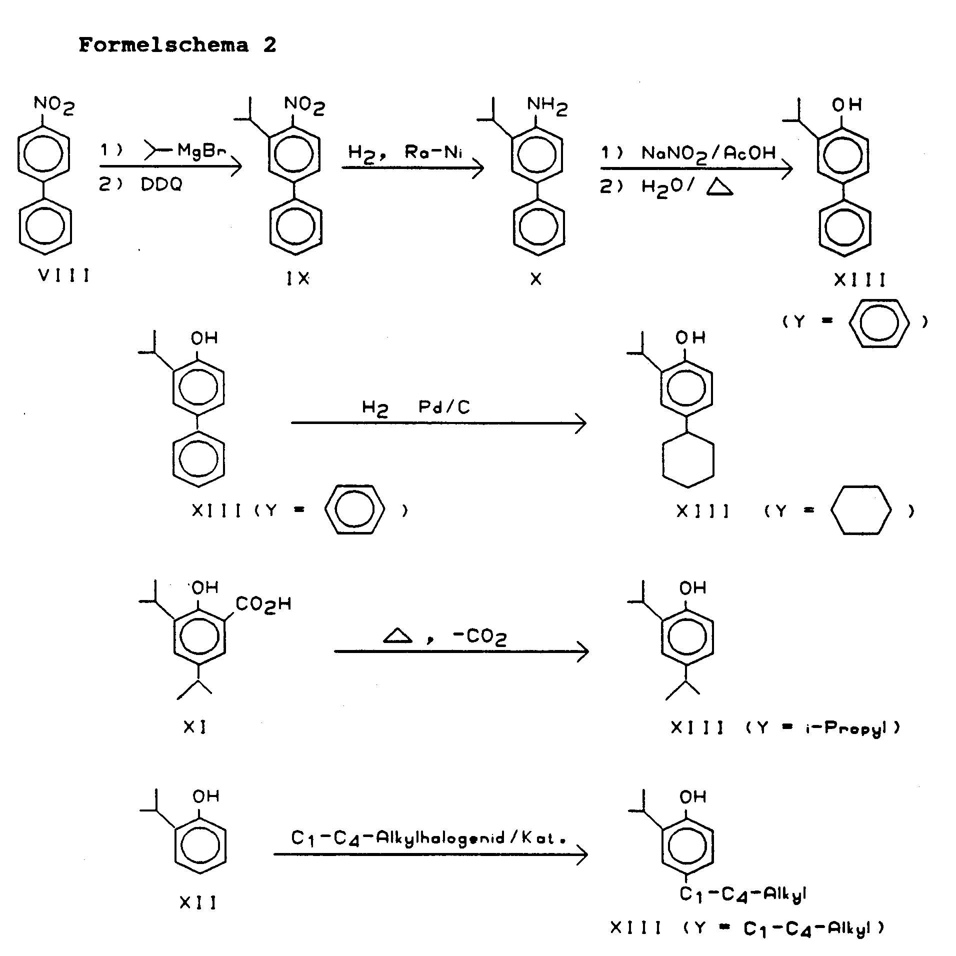

Die Lactone der Formel I können nach üblicher Methode (vgl. z.B. EP-A-0216127 und DE-A-36 32 893) in die entsprechenden offenkettigen Dihydroxycarbonsäuren der Formel II, deren pharmakologisch verträgliche Salze mit Basen und deren pharmakologisch verträgliche Ester umgewandelt werden.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterin-Biosynthese (I.R. Sabine, 3-Hydroxy-3-methylglutaryl Coenzym A Reductase, CRC Press, 1983). Hohe Cholesterinspiegel werden mit einer Reihe von Erkrankungen wie z.B. koronarer Herzkrankheit oder Atherosklerose in Verbindung gebracht. Daher ist die Senkung erhöhter Cholesterinspiegel zur Vorbeugung und Behandlung solcher Erkrankungen ein therapeutisches Ziel. Ein Ansatzpunkt liegt in der Hemmung bzw. Verminderung der endogenen Cholesterinsynthese. Hemmstoffe der HMG-CoA-Reduktase blockieren die Cholesterin-Biosynthese auf einer frühen Stufe. Sie eignen sich daher zur Vorbeugung und Behandlung von Erkrankungen, die durch einen erhöhten Cholesterinspiegel verursacht werden. Eine Reduktion bzw. Verminderung der endogenen Synthese führt zu einer erhöhten Aufnahme von Cholesterin aus dem Plasma in die Zellen. Ein zusätzlicher Effekt läßt sich durch gleichzeitige Gabe Gallensäuren-bindender Stoffe wie Anionenaustauscher erzielen. Die erhöhte Gallensäurenausscheidung führt zu einer verstärkten Neusynthese und damit zu einem erhöhten Cholesterinabbau (M.S. Brown, P.T. Kovanen, J.L. Goldstein, Science 212, 628 (1981): M.S. Brown, J.L. Goldstein Spektrum der Wissenschaft 1985 (1), 96). Die erfindungsgemäßen Verbindungen sind Hemmstoffe der HMG-CoA-Reduktase. Sie eignen sich daher zur Hemmung bzw. Verminderung der Cholesterin-Biosynthese und damit zur Vorbeugung oder Behandlung von Erkrankungen, die durch erhöhte Cholesterinspiegel verursacht werden, insbesondere koronare Herzkrankheit, Atherosklerose, Hypercholesterinämie, Hyperlipoproteinämie und ähnliche Erkrankungen.

Die Erfindung betrifft daher auch pharmazeutische Präparate auf Basis der Verbindungen der Formel I bzw. der entsprechenden Dihydroxycarbonsäuren der Formel II, deren Salze und Ester, sowie die Verwendung dieser Verbindungen als Arzneimittel, insbesondere zur Behandlung der Hypercholesterinämie.

Die Verbindungen der Formel I bzw. die entsprechenden Säuren, Salze oder Ester werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 2500 mg, vorzugsweise jedoch im Dosisbereich 10 bis 500 mg.

Die erfindungsgemäßen Verbindungen können als Laktone der allgemeinen Formel I, in Form der freien Säuren derFormel II oder in Form pharmazeutisch annehmbarer Salze oder Ester zur Anwendung kommen, und zwar gelöst oder suspendiert in pharmakologisch unbedenklichen organischen Lösungsmitteln wie ein- oder mehrwertigen Alkoholen wie z.B. Ethanol oder Glycerin, in Triacetin, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon oder anderen pharmazeutisch annehmbaren Zusatzstoffen wie Stärke, Cyclodextrin oder Polysacchariden. Ferner können die erfindungsgemäßen Verbindungen kombiniert werden mit Zusatzstoffen, die Gallensäuren binden, insbesondere nicht toxische, basische Anionenaustauscherharze, die Gallensäuren in einer nichtresorbierbaren Form im Gastrointestinaltrakt binden. Die Salze der Dihydroxycarbonsäuren können auch als wäßrige Lösung verarbeitet werden.

Die HMG-CoA-Reduktase-Aktivität der Natriumsalze der erfindungsgemäßen Verbindungen der Formel II wurde in zwei Testsystemen bestimmt
**1) Inhibierung der HMG-CoA-Reduktase-Aktivität an solubilisierten Enzympräparationen aus Ratten-Lebermikrosomen**
Die HMG-CoA-Reduktase-Aktivität wurde an solubilisierten Enzympräparationen aus Lebermikrosomen von Ratten gemessen, die nach Umstellung im Tag-Nacht-Rhythmus mit Cholestyramin (®Cuemid) induziert wurden. Als Substrat diente (S,R) ¹⁴C-HMG-CoA, die Konzentration von NADPH wurde während der Inkubation durch ein regenerierendes System aufrechterhalten. Die Abtrennung von ¹⁴C-Mevalonat von Substrat und anderen Produkten (z.B. ¹⁴C-HMG) erfolgte über Säulenelution, wobei das Elutionsprofil jeder Einzelprobe ermittelt wurde. Auf die ständige Mitführung von ³H-Mevalonat wurde verzichtet, weil es sich bei der Bestimmung um die Relativangabe der Hemmwirkung handelt. In einer Versuchsreiche wurden jeweils die enzymfreie Kontrolle, der enzymhaltige Normalansatz (= 100 %) und solche mit Präparatezusätzen zusammen behandelt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt. Nach der oben beschriebenen Methode wurden von den erfindungsgemäßen Verbindungen z.B. folgende Hemmwerte auf die HMG-CoA-Reduktase ermittelt (IC₅₀- (mol/l); molare Konzentration der Verbindung pro Liter, die für eine 50 %ige Hemmung erforderlich ist)

**Tabelle 1**

| Beispiel | IC₅₀ (mol/l) |
|---|---|
| 8a | 2,3 · 10⁻⁹ |
| 8b | > 10⁻⁷ |
| 8c | 1,7 · 10⁻⁸ |
| 8d | 2,3 · 10⁻⁸ |
| 8e | 5,2 · 10⁻⁹ |
| 8f | 4,8 · 10⁻⁹ |
| 8g | 3,6 · 10⁻⁸ |

**2) Supprimierung bzw. Inhibierung der HMG-CoA-Reduktase in HEP G2-Zellkulturen (einer Human-Hepatoma-Zell-Linie)**
Es erfolgte eine Bestimmung der Inhibition des Einbaus von ¹⁴C-Natriumacetat in Cholesterin.
Monolayers von HEP G2-Zellen in RPMJ 1640 Medium mit 10 % von Lipiden befreitem fötalem Kälberserum wurden 1 Stunde mit verschiedenen Konzentrationen der Natriumsalze der Dihydroxycarbonsäuren der Formel II präinkubiert. Nach Zugabe von ¹⁴C-markiertem Natriumacetat wurde die Inkubation 3 Std. fortgesetzt. Tritium-markiertes Cholesterin wurde als interner Standard zugefügt und ein Aliquot der Zellen alkalisch verseift. Die Lipide wurden mit Chloroform/Methanol 2:1 extrahiert. Nach Zugabe von Carrier-Cholesterin wurde das Lipidgemisch präparativ auf DC-Platten mit Chloroform/Aceton 9:1 getrennt. Die Cholesterin-Zone wurde durch Anfärben mit Joddampf sichtbar gemacht, außerdem mit einem DC-Radioscanner detektiert und dann abgeschabt. Die Menge ¹⁴C-Cholesterin wurde szintigraphisch bestimmt. In einem anderen Aliquot der Zell-Monolayers wurde (zur Berechnung der ¹⁴C-Cholesterinbiosynthese pro mg Zell-Protein) das Zellprotein gemessen. Dieselbe Prozedur wurde mit Zellen der gleichen Kultur ohne Präinkubation mit einer Testverbindung durchgeführt (sogenannte "Lösungsmittelkontrolle"). Die Wirkstärke der Testverbindungen wurde bestimmt durch Vergleich des biosynthetisierten ¹⁴C-Cholesterins in Testläufen und bei "Lösungsmittelkontrolle". Die Wirkstärke wurde auf der Basis von Mevinolin-Natriumsalz als externer Standard berechnet. Die IC₅₀- und IC₇₀-Werte (IC₅₀ bzw. IC₇₀ (M) ist die molare Konzentration der Verbindung pro Liter, die für eine 50 bzw. 70 %ige Hemmung erforderlich ist) variierten etwas für unterschiedliche Zell-Chargen. Die Mittelwerte für Mevinolin-Natriumsalz waren IC₅₀ = 5 · 10⁻⁸M, IC₇₀ = 1.5 · 10⁻⁷M. Die gemessenen IC's für Testverbindungen (Natriumsalze der Dihydroxycarbonsäuren der Formel II) (Tab. 2) wurden um die Abweichung von Mevinolin-Natrium von seinem Durchschnittswert korrigiert. Mevinolin-Natrium wurde eine relative Wirkstärke von 100 zugewiesen.

**Tabelle 2**

| Beispiel | IC₅₀ (M) | IC₇₀ (M) | relative Wirkstärke |
|---|---|---|---|
| 8a | 2,7 · 10⁻⁸ | 7 · 10⁻⁸ | 185 (214) |
| 8b | ∼ 10⁻⁵ | | < 1 |
| 8c | 9 · 10⁻⁸ | | 56 |
| 8d | 9,5 · 10⁻⁸ | | 53 |

Durch die folgenden Beispiele soll die Synthese der erfindungsgemäßen Verbindungen I weiter illustriert werden.

### Beispiel 1

### Synthese von 4(R)-Hydroxy-6(S)-[(2,4-diisopropyl-6-p-fluorphenyl)-phenoxymethyl]tetrahydro-2H-pyran-2-on

### (Formel I, X=O, Y=i-Pr, Z=H)

### Beispiel 1.1

### 2,4-Diisopropylphenol (Formel XIII, Y=i-Pr)

Das Gemisch aus 145 g (0,65 Mol) 3,5-Diisopropyl-2-hydroxybenzoesäure (XI), 540 ml (588 g, 4,55 Mol) Chinolin und 7,5 g (0,024 Mol) Kupferchromit (2CuO·Cr₂O₃) wird 2 Stunden bei 190°C (225°C Außentemperatur) gerührt. Man kühlt auf ca. 10°C, säuert unter weiterer Kühlung mit ca. 1 l halbkonzentrierter Salzsäure auf pH 1 bis 2 an, extrahiert mit Toluol und wäscht das Extrakt mit 2 N Salzsäure, dann mit Wasser und dann mit NaHCO₃-Lösung. Man trocknet, filtriert, engt ein und destilliert im Hochvakuum. Man erhält 105 g der Titelverbindung XIII als hellgelbes Öl, Sdp. 81 bis 84°C/0,2 Torr
- ¹H-NMR(CDCl₃):: δ 1,20 (6H,d); 1,25 (6H,d); 3,00 (2H,2xhept.); 4,10 (1H,s,br); 6,50-7,00 (3H,m)

### Beispiel 1.2

### 2,4-Diisopropyl-6-bromphenol (Formel XIV, Y=i-Pr)

Zur 95°C warmen Lösung von 102,3 g (0,57 Mol) 2,4-Diisopropylphenol in 900 ml Eisessig gibt man 1 g Eisenpulver, dann tropfenweise 101 g (32,2 ml, 0,63 Mol) Brom innerhalb 90 Minuten. Man rührt noch 1 Stunde bei 100°C, kühlt ab, verteilt das Reaktionsgemisch zwischen Toluol und Wasser und wäscht die Toluolphase mit NaHCO₃-Lösung. Man trocknet, filtriert, engt ein und destilliert den Rückstand im Hochvakuum. Man erhält 125 g der Titelverbindung XIV als hellgelbes Öl, Sdp. 85°C/0,15 Torr
- ¹H-NMR(CDCl₃):: δ 1,20 (6H,d); 1,25 (6H,d), 2,80 (1H,hept.); 3,25 (1H,hept.); 5,33 (1H,s); 6,87-7,20 (2H,m)
- MS (70eV):: m/e = 256/258 (M⁺), 241/243 (M⁺-CH₃)

### Beispiel 1.3

### 1-Benzyloxy-2,4-diisopropyl-6-brombenzol (Formel XV, Y=i-Pr)

Die Suspension von 166,5 g (1,2 Mol) Kaliumcarbonat in 124 g (0,48 Mol) des obigen Bromphenols, 91,52 g (0,72 Mol) Benzylchlorid und 2 l 2-Butanon wird 24 Stunden zum Rückfluß erhitzt.
Man kühlt ab, saugt den anorganischen Feststoff ab, engt das Filtrat im Vakuum ein und verteilt den Rückstand zwischen Toluol und Wasser. Die Toluolphase wird mit gesättigter Kochsalzlösung gewaschen, getrocknet, filtriert und eingeengt. Der Rückstand wird mit Cyclohexan/Toluol 9:1 über Kieselgel chromatographiert. Man erhält 155 g der Titelverbindung XV als farbloses Öl.
Geringe Reste Benzylchlorid werden im Hochvakuum entfernt. Man kann die Reinigung auch durch Destillation erreichen (Sdp. 150 °C/0,15 Torr).
- ¹H-NMR(CDCl₃):: δ 1,18 (6H,d); 1,22 (6H,d), 2,80 (1H,hept.); 3,32 (1H,hept.); 4,90 (2H,s); 6,93-7,60 (7H,m)
- MS (70eV):: m/e = 346/348 (M⁺), 267, 254/256, 91

### Beispiel 1.4

### 1-Benzyloxy-2,4-diisopropyl-6-p-fluorphenyl-benzol (Formel XVIII, Y=i-Pr, Z=H)

Man stellt die Grignard-Verbindung XV (Y=i-Pr) aus 48,62 g (0,14 Mol) des Bromids aus Beispiel 1.3 und 3,53 g (0,147 Mol) Mg-Spänen in 120 ml absolutem THF her (∼ 60°C, 1 Stunde). Diese Grignard-Lösung wird rasch zur Lösung von 31,08 g (0,14 Mol) 4-Fluorjodbenzol und 3,23 g (2,8 mMol) Tetrakis(triphenylphosphin)palladium (O) in 140 ml absolutem THF gegeben. Die Innentemperatur steigt innerhalb von 15 Minuten auf 55 bis 60°C. Nach 7 Minuten bildet sich ein Niederschlag. Man rührt 1 Stunde bei 50 bis 58°C, läßt über Nacht bei Raumtemperatur stehen, verteilt zwischen Ether und 1 N Salzsäure, wäscht die Etherphase mit 1 N Salzsäure, dann mit Wasser und dann mit gesättigter NaHCO₃-Lösung. Man trocknet, filtriert und engt ein. Bei Bedarf reinigt man das Produkt durch Chromatographie mit Cyclohexan/Toluol 4:1 über Kieselgel oder durch Destillation (Sdp. 180 °C/0,3 Tor). Man erhält 49,3 g der Titelverbindung XVIII als farblosen Feststoff, Schmp. 65 bis 67°C
- ¹H-NMR(CDCl₃):: δ 1,30 (12H,d); 2,95 (1H,hept.); 3,45 (1H,hept.); 4,40 (2H,s); 6,90-7,80 (11H,m)
- MS (CI):: m/e = 363 (M+H⁺), 362 (M⁺), 285, 263

### Beispiel 1.5

### 2,4-Diisopropyl-6-p-fluorphenyl-phenol (Formel III, Y=i-Pr, Z=H)

Zur Lösung von 49,3 g (0,136 Mol) des Benzylethers XVIII aus Beispiel 1.4 in 1 l Essigester und 100 ml Eisessig gibt man 4 g 10 % Pd auf Kohle und schüttelt 20 Minuten in einer Wasserstoffatmosphäre (lebhafte H₂-Aufnahme). Man filtriert den Katalysator ab, engt ein, nimmt den Rückstand mehrmals in Toluol auf und engt jeweils im Vakuum ein. Man erhält 34,4 g der Titelverbindung III als farbloses Öl, Sdp. 115 °C/0,1 Torr.
- ¹H-NMR(CDCl₃,270MHz):: δ 1,25 (6H,d); 1,29 (6H,d); 2,87 (1H,hept.); 3,31 (1H,hept.); 4,95 (1H,s,br); 6,88(1H,d); 7.08 (1H,d) 7,18 (2H,m); 7,45 (2H,m).
- MS (70eV):: m/e = 272 (M⁺), 257 (M⁺-CH₃)

### Beispiel 1.6

### 6(S)-{(2,4-Diisopropyl-6-p-fluorphenyl)-phenoxymethyl}-3,4,5,6-tetrahydro-2(R,S)-methoxy-4(R)-(t-butyldiphenylsilyloxy)-2H-pyran (Formel V, Y=i-Pr, Z=H)

Zur Suspension von 27.6 g (0,2 Mol) Kaliumcarbonat und einer Spatelspitze Hydrochinon in 250 ml abs. DMSO gibt man 27,2 g (0,1 Mol) des Phenols aus Beispiel 1.5. Man rührt 1 Std. bei Raumtemperatur und gibt dann die Lösung von 56 g (0,11 Mol) des Laktoletherjodids IV (Herstellung siehe EP-A 0 216 127, R₇ = t-Butyldiphenylsilyl) in 250 ml abs. DMSO zu. Man rührt 4 Std. bei 50-55 °C Innentemperatur. DC (Kieselgel, 1. Entwicklung mit Cyclohexan/Essigester 9:1, 2. Entwicklung mit Cyclohexan/Essigester 15:1) zeigt vollständigen Umsatz des Jodids IV (R_{f} 0,5), etwas restliches Ausgangsphenol (R_{f} 0,7), hauptsächlich Produkt der Formel V (R_{f} 0,6) an. Man läßt abkühlen und verteilt das Reaktionsgemisch zwischen Ether und halbgesättigter Kochsalzlösung. Die wäßrige Phase wird nochmals mit Ether extrahiert. Die vereinigten organischen Phasen werden mit Kochsalzlösung gewaschen, über MgSO₄ getrocknet, filtriert und eingeengt. Das Rohprodukt wird mit Toluol/Cyclohexan 2:1, dann 100 % Toluol, dann Toluol/Essigester 30:1 über Kieselgel chromatographiert. Man erhält 51 g der Titelverbindung als farbloses Harz.
- ¹H-NMR(CDCl₃):: δ 1,10 (9H,s); 1,28 (12H,d), 1,4-2,2 (4H,m); 2,93 (2H,2xhept.); 3,40 (2H,m); 3,52 (3H,s); 3,97-4,40 (2H,qui+m); 4,87 (1H,dd); 6,87-7,90 (16H,m)
- MS (CI):: m/e = 654 (M⁺), 597 (M⁺-tert.-Bu), 539, 519, 323, 283, 135, 127

### Beispiel 1.7

### 6(S)-{2,4-Diisopropyl-6-p-fluorphenyl)-phenoxymethyl}-3,4,5,6-tetrahydro-2-(R,S)-hydroxy-4(R)-(t-butyldiphenylsilyloxy)-2H-pyran (Formel VI, Y=i-Pr, Z=H)

Die Lösung von 40,2 g (61,4 mMol) des Laktolethers aus Beispiel 1.6 in 3 l THF, 3 l Wasser und 4,2 l Eisessig wird 24 Std. bei 80-85 °C (Außentemperatur) gerührt. Die Lösungsmittel werden im Vakuum entfernt und der Rückstand 3 mal mit Toluol im Vakuum abgeraucht. Chromatographie mit Cyclohexan/Essigester 12:1 durch 2 l Kieselgel gibt 33,4 g (Ausbeute 85 %) der Titelverbindung als farbloses amorphes Pulver.
- MS (FAB):: m/e 640 (M⁺), 519, 367, 323, 283, 271, 257

### Beispiel 1.8

### 6(S)-{2,4-Diisopropyl-6-p-fluorphenyl)-phenoxymethyl}-3,4,5,6-tetrahydro-4(R)-(t-butyldiphenylsilyloxy)-2H-pyran-2-on (Formel VII, Y=i-Pr, Z=H)

Zur Lösung von 33,4 g (52,1 mMol) des Lactols aus Beispiel 1.7 und 19,25 g (52,1 mMol) Tetrabutylammoniumjodid in 2,5 l absolutem Methylenchlorid gibt man unter Rühren und Kühlung 46,9 g (208,4 mMol) N-Jodsuccinimid. Man rührt unter Lichtausschluß unter Stickstoff 1 Stunde bei 10°C, 20 Stunden bei Raumtemperatur. Man wäscht die Reaktionslösung mit Wasser, dann 2mal mit NaHSO₃-Lösung, dann mit gesättigter NaCl-Lösung, trocknet, filtriert und engt ein. Der Rückstand wird in wenig Methylenchlorid gelöst und mit Cyclohexan/Essigester 92:8 durch Kieselgel filtriert. Man erhält 32,1 g der Titelverbindung als farbloses Harz.
- ¹H-NMR(CDCl₃,270MHz):: δ 1,06 (9H,s); 1,23 (6H,d); 1,26 (6H,d); 1,59 (2H,m); 2,41 (1H,dd); 2,59 (1H,dm); 2,90 (1H,hept.); 3,36 (1H,hept.); 3,48 (2H, AB von ABX); 4,29 (1H,qui); 4,80 (1H,m); 6,96 (1H,d); 7,03 (2H,m); 7,10 (1H,d); 7,36-7,52 (8H,m); 7,58-7,73 (4H,m)
- MS (70eV,70°C):: m/e = 638 (M⁺), 581 (M⁺-tert.-Bu), 539 (581 - Propen), 283, 199

### Beispiel 1.9

### 4(R)-Hydroxy-6(S)-[(2,4-diisopropyl-6-p-fluorphenyl)-phenoxymethyl]-tetrahydro-2H-pyran-2-on (Formel I, X=O, Y=i-Pr, Z=H)

Zur Lösung aus 31,0 g (48,5 mMol) der Silylverbindung aus Beispiel 1.8 in 1.5 l Tetrahydrofuran (über bas. Al₂O₃ filtriert) gibt man 11,65 g (194 mMol) Eisessig, gefolgt von 45,92 g (145,5 mMol) Tetrabutylammoniumfluorid-Trihydrat. Man rührt 20 Std. bei Raumtemperatur. Die Lösungsmittel werden im Vakuum entfernt und der Rückstand wird sofort zwischen Ether und Wasser verteilt. Die wäßrige Phase wird noch zweimal mit Ether extrahiert. Die vereinigten organischen Phasen werden mit gesättigter Kochsalzlösung gewaschen, über MgSO₄ getrocknet, filtriert und eingeengt.
Der Rückstand wird in Toluol aufgenommen und im Vakuum eingeengt. Das Rohprodukt wird mit Cyclohexan/Essigester 1:1 durch 2 kg Kieselgel chromatographiert.
Man erhält 15,7 g (Ausbeute 81 %) der Titelverbindung als farblosen Feststoff, Schmp. 145-147 °C.
- ¹H-NMR(CDCl₃,270MHz):: δ 1,25 und 1,27 (12H,2xd); 1,67 (1H,s,br.); 1,76 (1H,dtd); 1,87 (1H,ddd); 2,58 (1H,ddd); 2,69 (1H,dd); 2,91 (1H,hept); 3,39 (1H,hept), 3,54 (2H,AB von ABX); 4,38 (1H,qui), 4,68 (1H,m); 6,97 (1H,d); 7,10 (3H,d+m); 7,51 (2H,m)
- MS (FAB):: m/e = 400 (M⁺), 257

### Beispiel 2

### Synthese von 4(R)-Hydroxy-6(S)-[(2-isopropyl-4-tert.-butyl-6-p-fluorphenyl)-phenoxymethyl]tetrahydro-2H-pyran-2-on

### (Formel I, X=O, Y=tert.-But, Z=H)

### Beispiel 2.1

### 2-Isopropyl-4-tert.-butyl-phenol (Formel XIII, Y=tert-Bu)

Zur Suspension von 70 g (0,3 Mol) Zirkoniumtetrachlorid in 100 ml absolutem CH₂Cl₂ tropft man bei -5 bis 0°C unter Stickstoff langsam die Lösung von 34 g (0,25 Mol) o-Isopropylphenol (Formel XII) und 22 g (0,26 Mol) tert.-Butylmethylether in 150 ml absolutem CH₂Cl₂. Man rührt 1 Stunde bei 0°C. DC (100 % Toluol) zeigt ca. 50 % Umsatz an. Man gibt auf einmal rasch weitere 70 g (0,3 Mol) ZrCl₄ zu und rührt die braune Suspension 15 Minuten bei 0°C. DC zeigt nun > 95 % Umsatz und keinerlei Verunreinigungen an.* Man tropft bei -10 bis 0°C unter sehr guter Kühlung langsam 500 ml gesättigte NaHCO₃-Lösung zu (sehr exotherm). Es entsteht ein farbloser Feststoff, der das mechanische Rühren sehr erschwert. Man trennt die organische Phase ab, trocknet und engt im Vakuum ein. Bei Bedarf chromatographiert man das Produkt mit Cyclohexan/Toluol 1 : 2 durch 800 g Kieselgel oder man destilliert im Vakuum. Man erhält 43,1 g der Titelverbindung XIII als farblosen Feststoff, Schmp. 55 bis 57°C, Sdp. 134 bis 135°C/12 Torr
- ¹H-NMR(CDCl₃):: δ 1,27 (6H,d); 1,28 (9H,s); 3,17 (1H,hept.); 4,61 (1H,s); 6,62 (1H,d); 7,05 (1H,dd); 7,17 (1H,d)
- MS (70eV):: m/e = 192 (M⁺)

* Läßt man das Reaktionsgemisch 10 Stunden bei Raumtemperatur unter Stickstoff stehen, so zeigt DC wieder ca. 30 % Ausgangsmaterial und zahlreiche Nebenprodukte an.

### Beispiel 2.2

### 2-Isopropyl-4-tert.-butyl-6-bromphenol (Formel XIV, Y=tert.-Bu)

Zur Lösung von 65,8 g (0,34 Mol) des Phenols XIII aus Beispiel 2.1 in 375 ml CCl₄ tropft man 18 ml (55,8 g, 0,35 Mol) Brom. Man kontrolliert mit DC (Cyclohexan/Ethylacetat 5:1, R_{f} XIII 0,37, XIV: 0,33) den vollständigen Umsatz des Ausgangsmaterials, nimmt in Ether auf und wäscht 2mal mit Na₂S₂O₃-Lösung, 1mal mit gesättigter NaCl-Lösung. Man trocknet, engt ein und destilliert im Hochvakuum. Man erhält 86,1 g der Titelverbindung XIV als blaßgelbes Öl, Sdp. 105 bis 106°C/1 Torr
- ¹H-NMR(CDCl₃):: δ 1,25 (6H,d); 1,29 (9H,s); 3,47 (1H,hept.); 6,17 (1H,br.); 7,09 (1H,d); 7,24 (1H,d)
- MS (70eV):: m/e = 270/272 (M⁺)

### Beispiel 2.3

### 2-Isopropyl-4-tert.-butyl-6-jodphenol (Formel XIX, Y=tert.-Bu)

Zur Lösung von 19,2 g (0,1 Mol) des Phenols XIII aus Beispiel 2.1 in 150 ml 50 %iger wäßriger Ethylamin-Lösung und 120 ml Ethanol tropft man bei 20 bis 25°C die Lösung von 30,4 g (0,12 Mol) Jod und 40,0 g (0,24 Mol) Kaliumjodid in 120 ml Wasser. Man rührt 1 Stunde bei Raumtemperatur, nimmt in Ether auf, wäscht 2mal mit Na₂S₂O₃-Lösung, dann mit gesättigter NaClLösung, trocknet, engt im Vakuum ein, nimmt in Toluol auf und engt bei < 25°C im Vakuum ein. Man erhält 26,0 g der Titelverbindung XIX als Öl.
- ¹H-NMR(CDCl₃):: δ 1,15-1,50 (15H,s+d); 3,06 (1H,hept.); 4,60 (1H,s,br.); 6,86 (1H,s); 7,73 (1H,s)
- MS (70eV, <50°C):: m/e = 318 (M⁺), 303 (M⁺-CH₃), 275, 177, 161

### Beispiel 2.4

### 1-Benzyloxy-2-isopropyl-4-tert.-butyl-6-brombenzol (Formel XV, Y=tert.-Bu)

erhält man analog zu Beispiel 1.3 aus der Verbindung XIV, Beispiel 2.2.
Farblose Kristalle, Schmp. 47 bis 49°C.
- ¹H-NMR(CDCl₃):: δ 1,23 (6H,d); 1,48 (9H,s); 3,33 (1H,hept.); 5,12 (2H,s); 7,02 (1H,s); 7,44 (6H,s,br.)
- MS (70eV):: m/e = 360/362 (M⁺), 268/270, 91

### Beispiel 2.5

### 1-Benzyloxy-2-isopropyl-4-tert.-butyl-6-p-fluorphenyl-benzol (Formel XVIII, Y=tert.-Bu, Z=H)

erhält man analog zu Beispiel 1.4 aus der entsprechenden Grignard-Verbindung XVI.
Farbloser Feststoff, Schmp. 126 bis 128°C.
- ¹H-NMR(CDCl₃):: δ 1,1-1,3 (15H,s+d); 3,38 (1H,hept.); 5,16 (2H,s); 6,83 (1H,s); 7,0-7,7 (10H,m)
- MS (70eV):: m/e = 376 (M⁺), 282, 91

### Beispiel 2.6

### 2-Isopropyl-4-tert.-butyl-6-p-fluorphenyl-phenol (Formel III, Y=tert.-Bu, Z=H)

erhält man analog zu Beispiel 1.5 aus der Verbindung XVIII aus Beispiel 2.5.
Farbloser Feststoff, Schmp. 109 bis 111°C.
- ¹H-NMR(CDCl₃):: δ 1,15 (9H,s); 1,23 (6H,d); 3,16 (1H,hept.); 4,65 (1H,s); 6,80 (1H,s); 6,9-7,4 (5H,m)
- MS (70eV):: m/e = 286 (M⁺), 271 (M⁺-CH₃), 229

### Beispiel 2.7

### 2-Isopropyl-4-tert.-butyl-6-p-fluorphenyl-phenol (Formel III, Y=tert.-Bu, Z=H) durch direkte Kupplung des Jodids XIX mit dem Grignard-Reagenz aus p-Bromfluorbenzol XX

Zur Lösung von 25,7 g (81 mMol) des Jodphenols aus Beispiel 2.3 in 150 ml absolutem THF gibt man 1,87 g (1,6 mMol) Tetrakis(triphenylphosphin)palladium (O) und rührt 30 Minuten bei Raumtemperatur. Man gibt auf einmal das Grignard-Reagenz aus 42,6 g (243 mMol) 4-Bromfluorbenzol und 6,2 g (255 mMol) Mg-Spänen in 170 ml THF zu. Dabei steigt die Innentemperatur auf ca. 50°C. Man hält 3 Stunden auf 55°C, wobei sich ein farbloser Feststoff (Magnesiumjodid) abscheidet.* Man nimmt das Reaktionsgemisch in Ether auf, wäscht 2mal mit 1N Salzsäure, 1mal mit Wasser, 1mal mit gesättigter Natriumhydrogencarbonatlösung, trocknet und engt im Vakuum ein. Der Rückstand wird mit Cyclohexan/Ethylacetat 9 : 1 über 1 kg Kieselgel chromatographiert. Die XIII, XIX, III enthaltenden Fraktionen werden gemeinsam eingeengt. Der Rückstand wird in der Mindestmenge n-Pentan gelöst. Im Tiefkühlschrank kristallisieren 9,8 g reines III. Schmp. und Spektrum dieses Materials waren identisch mit den in Beispiel 2.6 angegebenen.
* Der Reaktionsverlauf kann nicht mit DC verfolgt werden, da das Jodid XIX, das Kupplungsprodukt III und das Phenol XIII, das während der Kupplung als Nebenprodukt entsteht, in allen gebräuchlichen Laufmitteln cochromatographieren. Geeignete Trennbedingungen: HPLC auf 250x4,6 Säule RP 18,5 µm Nucleosil, 64 % (CH₃OH + 0,1 % NH₄OAc)/36 % H₂O, 1,2 ml/min, 40°C, UV-Detektion 254 nm.

### Beispiele 2.8 bis 2.11

### 4(R)-Hydroxy-6(S)-[2-isopropyl-4-tert.-butyl-6-p-fluorphenyl)-phenoxymethyl]-tetrahydro-2H-pyran-2-on

### (Formel I, X=O, Y=tert.-Bu, Z=H)

erhält man aus dem Phenol III (Beispiel 2.6 oder 2.7) analog zu den Beispielen 1.6 bis 1.9.
Farbloser Feststoff, Schmp. 178-179 °C
- ¹H-NMR (CD₂Cl₂):: δ 1,13-1,20 (15H,m), 2,02 (1H,s,br.), 2,10-2,16 (2H,m), 2,71 (2H,AB von ABX), 3,24 (1H, hept.), 4,22 (2H;AB von ABX), 4,50 (1H,s,br.), 5,03-5,13 (1H,m), 6,79 (1H,s), 6,98-7,07 (3H,m), 7,19-7,25 (2H,m)
- MS (70 ev):: m/e = 414 (M⁺), 359
- IR (KBr):: 3560/3460 (OH), 1745 (C=O), 1500, 1235, 1220 cm⁻¹

### Beispiel 3:

### Synthese von 4(R)-Hydroxy-6(S)-[(2-isopropyl-4,6-di-p-fluorphenyl)-phenoxymethyl]tetrahydro-2H-pyran-2-on

### Formel I, X=O,

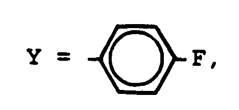

Z=H

### Beispiel 3.1

### 2-Isopropyl-4,6-dijod-phenol (Formel XXI)

Zur Lösung von 40,8 g (0,3 Mol) o-Isopropylphenol in 630 ml 50%iger wäßriger Ethylamin-Lösung und 525 ml ethanol tropft man innerhalb von 10 Min. bei 0 - 15°C die Lösung von 160 g (0,63 Mol) Jod und 209 g (1,26 Mol) Kaliumjodid in 300 ml Wasser. Man rührt 20 Min. bei Raumtemperatur und gießt das Reaktionsgemisch auf 200 ml gesättigte Na₂S₂O₃-Lösung plus 600 ml Wasser. Man extrahiert mit 3 x 500 ml Ether, wäscht die vereinigten Extrakte mit E./2 N Salzsäure, dann mit Wasser. Man trocknet über MgSO₄, dekantiert auf frisches MgSO₄, filtriert und engt im Vakuum bei < 20°C ein. Man setzt 100 ml Toluol zu und engt im Vakuum bei < 20°C ein. Dieser Abrauchvorgang mit Toluol wird einmal im Wasserstrahlvakuum, einmal im Hochvakuum wiederholt. Man erhält 99,0 g der Titelverbindung als rotes Öl. Im NMR, MS und DC sind keine Verunreinigungen zu erkennen.
- ¹H-NMR (CDCl₃):: δ 1,2 (6H,d), 3,2 (1H,hept.), 3,5 (1H,s), 7,35 (1H,d), 7,75 (1H,d)
- MS (70 eV):: m/e 388 (M⁺), 373 (M⁺-CH₃), 246 (M⁺-CH₃J)

### Beispiel 3.2

### 2-Isopropyl-4,6-di-p-fluorphenyl-phenol (Formel III′, Z=H)

Zur Lösung von 125 g (0,32 Mol) des Dijodids XXI aus Beispiel 3.1 und 5 g (7,1 mMol) Bis-(triphenylphosphin)palladium(II)-chlorid (Aldrich) in 300 ml absol. THF tropft man unter Argon und Eiskühlung die Grignard-Lösung aus 219 g (1,25 Mol) p-Bromfluorbenzol und 31,3 g (1,3 Mol) Magnesiumspänen in 600 ml absol. THF (25 - 30°C Innentemperatur). Man rührt 5 Std. bei 40 - 50°C, gibt dann weitere 2,5 g (PPh₃)₂PdCl₂ zu und rührt über Nacht bei ca. 45°C. Man kühlt auf 0°C und tropft 50 ml Wasser (exotherme Reaktion) so zu, daß die Innentemperatur unter 25°C bleibt. Es bildet sich ein zäher, schleimiger Niederschlag. Man tropft bei < 25°C 300 ml halbkonz. Salzsäure zu (Niederschlag löst sich auf, pH∼1). Das Gemisch wird mehrmals mit Ether extrahiert. Die vereinigten Extrakte werden mit 1N Salzsäure, dann mit gesättigter NaHCO₃-Lösung und dann mit gesättigter Kochsalzlösung gewaschen und anschließend getrocknet und eingeengt. Man erhält ein schwarzes, zähes Öl, das zunächst mit 4 l Cyclohexan/Toluol (4:1), dann mit 10 l Cyclohexan/Toluol (3:1), dann mit Cyclohexan/ Toluol (2,5:1) durch 1 kg Kieselgel 70 - 200 mµ chromatographiert wird.

Man eluiert zunächst 10,2 g eines farblosen Feststoffes, bei dem es sich gemäß NMR (nur aromatische Protonen), MS: 360, 342, 284, 266 (Basispeak), 248 und Analyse (C+H+F=100 %) um ein Oligomerengemisch handelt, das aus der Grignard-Verbindung XX (Z=H) entsteht.
m/e = 266 entspricht 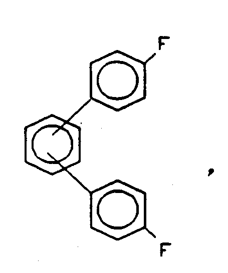
248 ist 266-F+H, 284 ist 266+F-H, 360 ist 266+C₆H₄F-H, 342 ist 360-F+H.

Man eluiert dann 2,1 g eines Monokupplungsprodukts, bei dem es sich um 2-Isopropyl-4-p-fluorphenyl-6-jod-phenol XIX′ (Y = p-Fluorphenyl) handelt.
MS (70 eV): m/e= 356 (M⁺), 341 (M⁺-CH₃), 214
Schließlich eluiert man 45,1 g der Titelverbindung III′ als zähes, farbloses Öl, das bei längerem Stehen bei Raumtemperatur kristallisiert.
- ¹H-NMR(CDCl₃):: δ 1,35 (6H,d); 3,4 (1H,hept.); 5,1 (1H,s); 6,8-7,6 (10H,m)
- MS (70eV):: m/e = 324 (M⁺), 309 (M⁺-CH₃)
DC (Toluol/Cyclohexan 1:2) R_{f}-Werte: Oligomerengemisch 0,61, Ausgangsmaterial XXI 0,53, Monojodid XIX′ 0,50, Produkt III′: 0,35

### Beispiele 3.3 bis 3.6

### 4(R)-Hydroxy-6(S)-[(2-isopropyl-4,6-di-p-fluorphenyl)-phenoxymethyl]-tetrahydro-2H-pyran-2-on

### (Formel I, X=O,

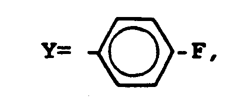

**Z=H)**
erhält man aus dem Phenol III′ (Beispiel 3.2) analog zu den Beispielen 1.6 bis 1.9.
Farbloser Feststoff, Schmp. 190-192 °C
- ¹H-NMR (CDCl₃, 270 MHz):: δ 1,31 (6H,2xd), 1,72-1,95 (3H,m), 2,66 (2H,AB von ABX), 3,47 (1H, hept.), 3,59 (2H,AB von ABX), 4,40 (1H,m), 4,70 (1H,m), 7,12 (4H,m), 7,28 (1H,d), 7,42 (1H,d). 7,55 (4H,m)
- MS (DCI):: m/e = 452 (M⁺), 437 (M⁺-CH₃), 129
- IR (KBr):: 3480 (OH), 1715 (C=O), 1510, 1255, 1220, 1200, 1160, 830 cm⁻¹

### Beispiel 4

### Synthese von 4(R)-Hydroxy-6(S)-[(2-isopropyl-4-phenyl-6-p-fluorphenyl)-phenoxymethyl]tetrahydro-2H-pyran-2-on

### (Formel I, X=O, Y = Phenyl, Z=H)

### Beispiel 4.1

### 2-Isopropyl-4-phenyl-nitrobenzol (Formel IX)

Zur Lösung aus 20,0 g (0.1 Mol) 4-Nitrobiphenyl VIII in 400 ml absolutem THF tropft man bei -70°C unter Stickstoff innerhalb von 3 Stunden die Grignard-Lösung aus 30,7 g (0.25 Mol) 2-Brompropan und 5,85 g (0.24 Mol) Magnesiumspänen in 300 ml absolutem THF. Man rührt noch 1 Stunde bei -70°C (DC: VIII vollständig umgesetzt) und tropft dann bei -40°C rasch die Lösung von 22,7 g (0.1 Mol) 2,3-Dichlor-5,6-dicyan-p-benzochinon (DDQ) in 200 ml absolutem THF zu. Man läßt auf Raumtemperatur erwärmen, rührt noch 1 Stunde und gießt auf 1,2 l Wasser. Man zieht das THF im Vakuum ab, extrahiert den wäßrigen Rückstand 2mal mit Essigester, wäscht die Extrakte gut mit Wasser, trocknet und engt ein. Chromatographie mit Cyclohexan/CH₂CH₂ 4:1 durch 1 kg Kieselgel ergibt 8,9 g der Titelverbindung IX als hellrotes Öl.
- ¹H NMR(CDCl₃):: δ 1,37 (6H, d); 3,58 (1H,hept.); 7,36-7.97 (8H,m)
- MS (70eV,50°C):: m/e = 241 (M⁺), 224, 174, 152

### Beispiel 4.2

### 2-Isopropyl-4-phenyl-anilin (Formel X)

13,1 g (54,3 mMol) der Nitroverbindung IX aus Beispiel 4.1 werden in der Lösung von 10 g Ammoniak in 400 ml Methanol gelöst. Man gibt unter Stickstoff 10 g Raney-Nickel zu, das dreimal mit Methanol gewaschen wurde. Man schüttelt die Suspension 2 Stunden bei Raumtemperatur und Normaldruck in einer Wasserstoffatmosphäre. Der Katalysator wird abfiltriert, das Filtrat eingeengt und der Rückstand mit 2 l Cyclohexan/Toluol 1 : 2, dann mit 5 l Toluol über 400 g Kieselgel chromatographier. Man erhält 11,2 g der Titelverbindung X als farbloses Öl.
- ¹H-NMR(CDCl₃):: δ 1,33 (6H,d); 3,00 (1H,hept.); 3,45 (2H,s,br.); 6,80 (1H,d); 7,2-7,7 (7H,m)
- MS (70eV):: m/e = 211 (M⁺), 196 (M⁺-CH₃)

### Beispiel 4.3

### 2-Isopropyl-4-phenyl-phenol (Formel XIII,

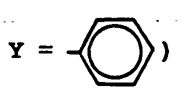

Zur Lösung von 11,2 g (53,1 mMol) des Amins X aus Beispiel 4.2 in 50 ml Eisessig tropft man bei 10 bis 12°C die Lösung von 4,26 g (62 mMol) Natriumnitrit in 50 ml Wasser. Das Diazoniumsalz fällt aus. Die Suspension ist schlecht rührbar. Nach 5 Minuten wird die Suspension langsam in die siedende Lösung von 32 ml konzentrierter Schwefelsäure in 65 ml Wasser gegossen. Man rührt noch 5 Minuten, kühlt dann ab und verteilt zwischen Toluol/Ether und gesättigter Kochsalzlösung. Die organische Phase wird 2mal mit gesättigter NaHCO₃-Lösung und 1mal mit Kochsalzlösung gewaschen, dann getrocknet und eingeengt. Chromatographie mit 100 % Toluol durch 500 g Kieselgel ergibt 4,9 g der Titelverbindung XIII als gelbes Öl.
- ¹H-NMR(CDCl₃):: δ 1,30 (6H,d); 3,25 (1H,hept.); 7,1-7,7 (8H,m)
- MS (70eV):: m/e = 212 (M⁺), 197 (M⁺-CH₃), 178

### Beispiel 4.4

### 2-Isopropyl-4-phenyl-6-brom-phenol (Formel XIV, Y = Phenyl)

erhält man analog zu Beispiel 2.2 aus dem Phenol XIII aus Beispiel 4.3.
- ¹H-NMR(CDCl₃):: δ 1,26 (6H,d); 3,40 (1H,hept.); 5,63 (1H,s); 7,2-7,7 (7H,m)
- MS (70eV):: m/e = 290/292 (M⁺), 275/277 (M⁺-CH₃), 196, 165

### Beispiel 4.5

### 1-Benzyloxy-2-isopropyl-4-phenyl-6-brombenzol (Formel XV, Y = Phenyl)

erhält man analog zu Beispiel 1.3 aus dem Phenol XIV (Beispiel 4.4) als farbloses Öl, das langsam kristallisiert.
- ¹H-NMR(CDCl₃):: δ 1,24 (6H,d); 3,45 (1H,hept.); 5,05 (2H,s); 6,95-7,70 (12H,m)
- MS (70eV):: m/e = 380/382 (M⁺), 91

### Beispiel 4.6

### 1-Benzyloxy-2-isopropyl-4-phenyl-6-p-fluorphenyl-benzol (Formel XVIII, Y = Phenyl, Z=H)

erhält man analog zu Beispiel 1.4 aus der entsprechenden Grignard-Verbindung XVI als farbloses Öl, das langsam kristallisiert.
- ¹H-NMR(CDCl₃):: δ 1,28 (6H,d); 3,52 (1H,hept.); 5,00 (2H,s); 6,95-7,70 (16H,m)
- MS (70eV):: m/e = 396 (M⁺)

### Beispiel 4.7

### 2-Isopropyl-4-phenyl-6-p-fluorphenyl-phenol (Formel III, Y = Phenyl, Z=H)

erhält man analog zu Beispiel 1.5 aus XVIII aus Beispiel 4.6 als farblosen Feststoff.
- ¹H-NMR(CDCl₃):: δ 1,35 (6H,d); 3,40 (1H,hept.); 5,10 (1H,s,br.); 6,85-7,45 (11H,m)
- MS (70eV):: m/e = 306 (M⁺), 291 (M⁺-CH₃)

### Beispiel 4.8 bis 4.11

### 4(R)-Hydroxy-6(S)-[(2-isopropyl-4-phenyl-6-p-fluorphenyl)-phenoxymethyl]tetrahydro-2H-pyran-2-on (Formel I, X=O, Y = Phenyl, Z=H)

erhält man aus dem Phenol (Beispiel 4.7) analog zu den Beispielen 1.6 bis 1.9.
Farbloser Feststoff, Schmp. 184-187 °C
- ¹NMR(CDCl₃):: δ 1,30 (6H,2xd); 1,7-2,0 (3H,m), 2,65 (2H,m), 3,50 (1H,hept.), 3,60 (2H,m), 4,40 (1H,m), 4,70 (1H,m), 7,1-7,6 (11H,m)
- MS (DCI):: m/e = 434 (M⁺), 491 (M⁺-CH₃)

### Beispiel 5

### Synthese von 4(R)-Hydroxy-6(S)-[2-isopropyl-4-cyclohexyl-6-p-fluorphenyl)-phenoxymethyl]tetrahydro-2H-pyran-2-on (Formel I, X=O, Y = Cyclohexyl, Z=H)

### Beispiel 5.1

### 2-Isopropyl-4-cyclohexyl-phenol (Formel XIII, Y = Cyclohexyl)

2,0 g 5 % Palladium auf Kohle, suspendiert in 50 ml Essigester werden 30 Minuten bei Raumtemperatur in einer Wasserstoffatmosphäre geschüttelt. Man gibt die Lösung von 31,2 (0,1 Mol) des Phenols XIII aus Beispiel 4.3 in 250 ml Essigester unter Sauerstoffausschluß zu und schüttelt 5 Stunden bei 50°C unter 5 kg/cm² Wasserstoffdruck. Der Reaktionsablauf kann gaschromatographisch verfolgt werden [1 m SP 1000 auf ®Chromosorb WAW 80 bis 100 mesh, 220°C, 1,0 kg/cm² N₂-Trägergas, tᵣₑₜ: XIII 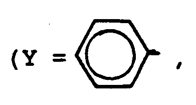
Ausgangsverbindung) 13,8 min, Produkt XIII 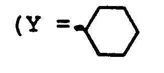
Cyclohexyl): 4,6 min].
Die GC-Analyse zeigt, daß ca. 90 % XIII (Y = Cyclohexyl) und mehrere Nebenprodukte, keines über 3 %, entstehen. Der Katalysator wird abfiltriert und der Rückstand aus Cyclohexan umkristallisiert. Man erhält 25,0 g der Titelverbindung XIII als farblosen Feststoff.
- ¹H-NMR(CDCl₃):: δ 0,8-1,2 (10H,m); 1,25 (6H,d), 3,00 (1H,m); 3,11 (1H,hept.); 4,24 (1H,s,br.); 6,50-7,10 (3H,m)
- MS (70eV):: m/e = 218 (M⁺), 203 (M⁺-CH₃)

### Beispiel 5.2

### 2-Isopropyl-4-cyclohexyl-6-jodphenol (Formel XIX, Y = Cyclohexyl)

erhält man analog zu Beispiel 2.3 aus dem Phenol XIII aus Beispiel 5.1.
- ¹H-NMR(CDCl₃):: δ 0,7-1,2 (10H,m); 1,26 (6H,d), 3,0-3,1 (2H,m); 4,60 (1H,s,br.); 6,88-7,40 (2H,m)
- MS (70eV):: m/e = 344 (M⁺), 329 (M⁺-CH₃)

### Beispiel 5.3

### 2-Isopropyl-4-cyclohexyl-6-p-fluorphenyl-phenol (Formel III, Y = Cyclohexyl, Z=H)

erhält man analog zu Beispiel 2.7 aus dem Jodphenol XIX aus Beispiel 5.2.
- ¹H-NMR(CDCl₃):: δ 0,7-1,2 (10H,m); 1,25 (6H,d), 3,0-3,2 (2H,m); 4,90 (1H,s,br.); 6,9-7,4 (6H,m)
- MS (70eV):: m/e = 312 (M⁺), 297 (M⁺-CH₃)

### Beispiele 5.4 bis 5.7

### 4(R)-Hydroxy-6(S)-[(2-isopropyl-4-cyclohexyl-6-p-fluorphenyl)-phenoxymethyl]tetrahydro-2H-pyran-2-on (Formel I, X=O, Y = Cyclohexyl, Z=H)

erhält man aus dem Phenol III aus Beispiel 5.3 analog zu den Beispielen 1.6 bis 1.9. Farbloser Feststoff, Schmp. 158 bis 160°C.
- ¹H-NMR(CDCl₃):: δ 0,8-1,1 (10H,m); 1,25 (6H,d); 1,68 (1H,s,br.); 1,75 (1H,m); 1,90 (1H,m); 2,55-2,70 (2H,m); 3,0-3,2 (2H,m); 3,55 (2H,m); 4,40 (1H,qui); 4,70 (1H,m); 7,0-7,5 (6H,m)
- MS (70eV):: m/e = 440 (M⁺)

### Beispiel 6

### Synthese von 4(R)-Hydroxy-6(S)-[(2,4-diisopropyl-6-p-fluorphenyl)-phenylthiomethyl]tetrahydro-2H-pyran-2-on (Formel I, X=S, Y=i-Pr, Z=H)

### Beispiel 6.1

### N,N-Dimethyl-[2,4-diisopropyl-6-p-fluorphenyl]thiocarbamidsäureester

3,6 g 50 %iges Natriumhydrid werden in 60 ml abs. DMF suspendiert. 21,76 g (80 mmol, 1 Äquiv.) 2,4-Diisopropyl-6-p-fluorphenyl-phenol (Beispiel 1.5) werden unter Eiskühlung eingetragen. Die Lösung wird 30 Min. bei Raumtemperatur gerührt und auf 0 °C abgekühlt. Die Lösung von 12,4 g (1,25 Äquiv.) Dimethylthiocarbamidsäurechlorid (Aldrich) in 20 ml DMF wird zugegeben und das Reaktionsgemisch wird 5 Std. bei 80-90 °C gerührt. Nach dem Abkühlen wird mit 500 ml Ether verdünnt, 2mal mit Wasser und 1mal mit Kaliumhydrogencarbonat-Lösung gewaschen, über Magnesiumsulfat getrocknet und das Lösungsmittel abgezogen. Der Rückstand wird aus Methanol umkristallisiert. Erhalten werden 25,6 g (Ausbeute 89 %) der Titelverbindung als Feststoff, Schmp. 182 °C
- MS:: m/e = 359 (M⁺)

### Beispiel 6.2

### S-[(2,4-Diisopropyl-6-p-fluorphenyl)phenyl]-N,N-dimethylthiocarbamat

25,0 g des Thiocarbamidsäureesters aus Beispiel 6.1 wurden unter Stickstoff 1 Std. auf 270-300 °C erhitzt. Nach dem Erhalten wurde der Rückstand in der Mindestmenge heißem n-Hexan gelöst, nach Zusatz von Aktivkohle 10 Min. unter Rückfluß gekocht und heiß filtriert. Aus dem Filtrat kristallisierten beim langsamen Abkühlen 20,0 g (80 % Ausbeute) der Titelverbindung als farblose Nadeln.
- MS:: m/e = 359 (M⁺)

### Beispiel 6.3

### 2,4-Diisopropyl-6-p-fluorphenyl-thiophenol (Formel III, X=S, Y=i-Pr, Z=H)

Zur Suspension von 3,2 g Lithiumaluminiumhydrid in abs. Ether tropft man unter Eiskühlung die Lösung von 19,7 g des Thiocarbamats aus Beispiel 6.2 in Ether. Man rührt 2 Std. bei Raumtemperatur und hydrolysiert unter Eiskühlung mit 2N Schwefelsäure (bis pH 3). Man extrahiert mehrmals mit Ether, trocknet über Magnesiumsulfat und zieht das Lösungsmittel ab. Man erhält 16,8 g der Titelverbindung als zähes Öl.
- MS:: m/e = 288 (M⁺), 273 (M⁺ - CH₃)

### Beispiel 6.4

### 6(S)-[(2,4-Diisopropyl-6-p-fluorphenyl)-phenylthiomethyl]-3,4,5,6-tetrahydro-2(R,S)-methoxy-4(R)-(t-butyldiphenylsilyloxy)-2H-pyran (Formel V, X=S, Y=i-Pr, Z=H, R⁷ = t-Butyl-diphenylsilyl)

Die Suspension aus 13,8 g (100 mMol) Kaliumcarbonat, 14,4 g (50 mMol) des Thiophenols aus Beispiel 6.3 und 20,4 g (40 mMol) des Laktoletherjodids IV (R⁷ = t-Butyl-diphenylsilyl, Herstellung siehe EP-A 0 216 127) in 300 ml abs. DMSO wurde 1 Std. bei 50 °C gerührt. Das abgekühlte Reaktionsgemisch wurde mit Wasser versetzt und 3mal mit Ether extrahiert. Die vereinigten organischen Phasen wurden mit Wasser, dann mit gesättigter Kochsalzlösung gewaschen, über Magnesiumsulfat getrocknet und im Vakuum eingeengt. Der Rückstand wurde mit Toluol/Essigester 95:5 über Kieselgel chromatographiert und ergab 21,4 g (80 % Ausbeute) der Titelverbindung als farbloses zähes Öl.
- MS (CI):: m/e = 670 (M⁺), 613 (M⁺-tert.-Bu)

### Beispiel 6.5

### 6(S)-[(2,4-Diisopropyl-6-p-fluorphenyl)-phenylthiomethyl]-3,4,5,6-tetrahydro-2(R,S)-hydroxy-4(R)-(t-butyldiphenylsilyloxy)-2H-pyran (Formel VI)

Die Lösung von 20,1 g (30 mMol) des Laktolethers V aus Beispiel 6.4 in 2 l THF, 1 l Wasser und 1 l Trifluoressigsäure wurde 1 Std. bei 50-60 °C gerührt. Nach Abkühlen auf Raumtemperatur wurden 1,5 kg Natriumacetat zugegeben. Die organischen Lösungsmittel wurden im Vakuum abgezogen. Der wäßrige Rückstand wurde mit 1 l gesättigter Kochsalzlösung versetzt und mehrmals mit Ether extrahiert. Die vereinigten organischen Extrakte wurden mit Wasser gewaschen und über Magnesiumsulfat getrocknet. Der Ether wurde abgezogen und der Rückstand mit Cyclohexan/Essigester 4:1 über Kieselgel chromatographiert. Man erhält 13,8 g (70 % Ausbeute) der Titelverbindung als farbloses, zähes Öl.
- MS (CI):: m/e = 656 (M⁺), 638 (M⁺ - H₂O), 581 (M⁺-t-Bu-H₂O)

### Beispiel 6.6

### 6(S)-[(2,4-Diisopropyl-6-p-fluorphenyl)-phenylthiomethyl]-3,4,5,6-tetrahydro-4(R)-(t-butyldiphenylsilyloxy)-2H-pyran-2-on (Formel VII)

Die Lösung von 13,0 g (19,8 mMol) des Laktols VI aus Beispiel 6.5, 7,4 g (20 mMol) Tetrabutylammoniumjodid und 22,5 g (100 mMol) N-Jodsuccinimid in 200 ml Methylenchlorid wurde 12 Std. bei Raumtemperatur gerührt. 500 ml Toluol wurden zugesetzt und das Methylenchlorid im Vakuum entfernt. Der Niederschlag wurde abgesaugt und mit Toluol gewaschen. Die vereinigten Filtrate wurden je einmal mit wäßriger Natriumthiosulfat-Lösung und gesättigter Kochsalzlösung gewaschen, über Magnesiumsulfat getrocknet, filtriert und im Vakuum eingeengt. Der Rückstand wurde mit Toluol/Essigester 10:1 über Kieselgel chromatographiert. Man erhält 11,6 g (90 % Ausbeute) der Titelverbindung als farbloses, zähes Öl.
- MS (70 ev, 70 °C):: m/e = 654 (M⁺), 597 (M⁺-t-Bu)

### Beispiel 6.7

### 4(R)-Hydroxy-6(S)-[(2,4-diisopropyl-6-p-fluorphenyl)-phenylthiomethyl]tetrahydro-2H-pyran-2-on

In Analogie zu Beispiel 1.9 erhält man aus 11,0 g des geschützten Laktons (Beispiel 6.6) 4,9 g (70 % Ausbeute) der Titelverbindung als zähes, farbloses Öl.
- ¹H-NMR (CDCl₃):: δ 1,25 (12H, 2xd), 1,65 (OH,s,br.), 1,7-1,9 (2H,m), 2,5-2,6 (2H,m), 2,75 (2H,m), 2,90 (1H, hept.), 3,40 (1H, hept.), 4,35 (1H,qui), 4,50 (1H,m), 7,0-7,5 (6H,m).
- MS (FAB):: m/e = 416 (M⁺)

### Beispiel 7

### Synthese von 4(R)-Hydroxy-6(S)-[(2-isopropyl-4,6-di-p-fluorphenyl)-phenylthiomethyl]tetrahydro-2H-pyran-2-on (Formel I, X=S, Y = p-Flurophenyl, Z=H)

Die Titelverbindung erhält man, indem man 2-Isopropyl-4,6-di-p-fluorphenyl-phenol (Bsp. 3.2) analog zu den Beispielen 6.1 bis 6.3 in 2-Isopropyl-4,6-di-p-fluorphenyl-thiophenol umwandelt und dieses analog zu den Beispielen 6.4-6.7 zur Titelverbindung umsetzt.
Farbloser, klebriger Feststoff, der beim Waschen mit n-Hexan kristalliner wird, Schmp. >60 °C
- ¹H-NMR (CDCl₃):: δ 1,3 (6H,d), 1,7-1,95 (3H,m), 2,5-2,7 (2H, m), 2,75 (2H,m), 3,4 (1H,hept.), 4,4 (1H,m), 4,6 (1H,m), 7,0-7,5 (10H,m).
- MS (FAB):: m/e = 468 (M⁺), 453 (M⁺-CH₃)
- IR (KBr):: 3480 (OH), 1715 (C=O)

### Beispiel 8

### Herstellung der Natriumsalze der offenkettigen Dihydroxycarbonsäuren (Formel II, Natriumsalz) aus den Laktonen der Formel I

### Beispiel 8a

### 3(R),5(S)-Dihydroxy-6-[2-(4-fluorphenyl)-4,6-diisopropylphenoxy]-hexansäure-Natriumsalz

Zur Lösung von 7,0 g (17,5 mMol) des Laktons aus Beispiel 1.9 in 800 ml abs. Ethanol tropft man unter Eiskühlung rasch 17,7 ml 1N Natronlauge. Man rührt 5 Min. unter Eiskühlung, dann 2 Stunden bei Raumtemperatur. Laut DC ist das Ausgangsmaterial vollständig umgesetzt. Lösungsmittel werden im Vakuum bei <30 °C Badtemperatur abgezogen. Der Rückstand wird 2mal in Ethanol gelöst und jeweils im Vakuum zur Trockne eingeengt, dann in Ether gelöst und im Vakuum zur Trockne eingeengt. Der Rückstand wird in Toluol suspendiert und im Vakuum zur Trockne eingeengt. Der Rückstand wird mit n-Pentan verrührt, dann abgesaugt und im Hochvakuum über Phosphorpentoxid und Kaliumhydroxid-Pastillen getrocknet. Man erhält 6,25 g der Titelverbindung als farbloses, amorphes Pulver. Beim Einengen der pentanischen Mutterlauge erhält man weitere 0,43 g amorphes Produkt. Schmp. 240-244 °C (Zers.). Die Zersetzungstemperatur hängt von der Aufheizgeschwindigkeit ab.

### Beispiel 8b

### 3(R),5(S)-Dihydroxy-6-[2-isopropyl-4-tert.-butyl-6-(4-fluorphenyl)-phenoxy]-hexansäure-Natriumsalz

wird analog zu Beispiel 8a aus dem Lakton aus Beispiel 2.11 erhalten.
Farbloses Pulver, Schmp. 256-258 °C (Zers.)

### Beispiel 8c

### 3(R),5(S)-Dihydroxy-6-[2-isopropyl-4,6-bis-(4-fluorphenyl)-phenoxy]-hexansäure-Natriumsalz

wird analog zu Beispiel 8a aus dem Lakton aus Beispiel 3.6 erhalten.
Farbloses Pulver, Schmp. 235-237 °C (Zers.)

### Beispiel 8d

### 3(R),5(S)-Dihydroxy-6-[2-isopropyl-4-phenyl-6-(4-fluorphenyl)-phenoxy-]-hexansäure-Natriumsalz

wird analog zu Beispiel 8a aus dem Lakton aus Beispiel 4.11 erhalten.
Fabloses Pulver, Schmp. 238-240 °C (Zers.)

### Beispiel 8e

### 3(R),5(S)-Dihydroxy-6-[2-isopropyl-4-cyclohexyl-6-(4-fluorphenyl)-phenoxy]-hexansäure-Natriumsalz

wird analog zu Beispiel 8a aus dem Lakton aus Beispiel 5.7 erhalten.
Farbloses Pulver, Schmp. 230-233 °C (Zers.)

### Beispiel 8f

### 3(R),5(S)-Dihydroxy-6-[2-(4-fluorphenyl)-4,6-diisopropylthiophenoxy]-hexansäure-Natriumsalz

wird analog zu Beispiel 8a aus dem Lakton aus Beispiel 6.7 erhalten.
Farbloses Pulver, Schmp. 230-234 °C (Zers.)

### Beispiel 8g

### 3(R),5(S)-Dihydroxy-6-[2-isopropyl-4,6-bis-(4-fluorphenyl)-thiophenoxy]-hexansäure-Natriumsalz

wird analog zu Beispiel 8a aus dem Lakton aus Beispiel 7 erhalten.

## Patentansprüche (Patentansprüche für folgende(n) Vertragsstaat(en): DE, GB, FR, IT, NL, SE, LI, CH, BE, AT, LU)

1. 6-Phenoxymethyl-4-hydroxy-tetrahydro-pyran-2-one und 6-Thiophenoxymethyl-4-hydroxy-tetrahydropyran-2-one der allgemeinen Formel I 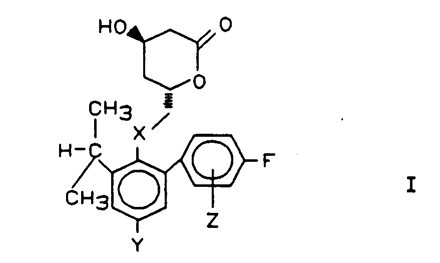 sowie die entsprechenden offenkettigen Dihydroxycarbonsäuren der Formel II 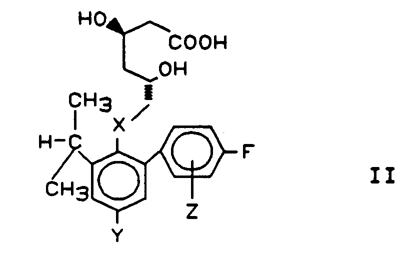 worin
X Sauerstoff oder Schwefel,
Y
a) einen geradkettigen oder verzweigten Alkylrest mit 3 bis 12 Kohlenstoffatomen
b) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 C-Atomen und
Z Wasserstoff oder einen geradkettigen oder verzweigten Alkylrest mit 1 bis 4 Kohlenstoffatomen,
bedeuten sowie deren pharmaklogisch verträgliche Salze mit Basen und deren pharmakologisch verträglich Ester.

2. Verbindungen gemäß Anspruch 1, dadurch gekennzeichnet, daß in den allgemeinen Formeln I bzw. II
X Sauerstoff
Y Isopropyl, tert.-Butyl, Cyclohexyl, Phenyl, p-Fluorphenyl
Z Wasserstoff bedeuten.

3. Verfahren zur Herstellung der Verbindungen der Formel I sowie der entsprechenden offenkettigen Dihydroxycarbonsäuren der Formel II, von deren pharmakologisch verträglichen Salzen mit Basen und deren pharmakologisch verträglichen Estern gemäß Anspruch 1, dadurch gekennzeichnet, daß man
a) entsprechend substituierte Phenole oder Thiophenole der Formel III 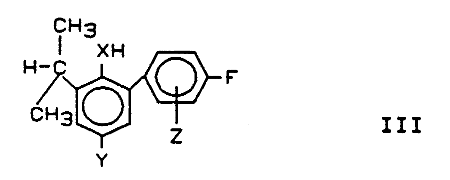 worin X, Y und Z die zu den Formeln I und II angegebenen Bedeutungen haben, mit dem optisch reinen Jodid der Formel IV 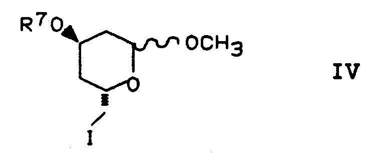 worin R⁷ eine gegen Basen und schwache Säuren stabile Schutzgruppe bedeutet, in die Lactolether der Formel V 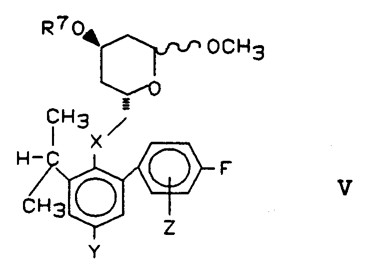 worin X, Y und Z die zu den Formeln I und II und R⁷ die zu Formel IV angegebenen Bedeutungen haben, überführt,
b) die Lactolether der Formel V zu den entsprechenden Lactolen der Formel VI 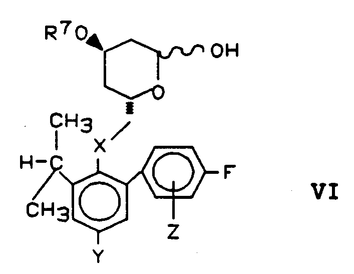 worin X, Y und Z die zu den Formeln I, II und R⁷ die zu Formel IV angegebene Bedeutungen haben, hydrolysiert,
c) die Lactole der Formel VI zu den entsprechenden Lactonen der Formel VII 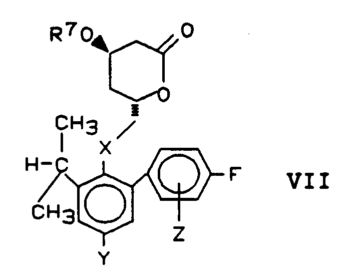 worin X, Y und Z die zu den Formeln I/II und R⁷ die zu Formel IV angegebenen Bedeutungen haben, oxydiert,
d) die erhaltenen geschützten Lactone der Formel VII in an sich bekannter Weise in Verbindungen der Formel I überführt und
e) gegebenenfalls die erhaltenen Verbindungen der Formel I in die entsprechenden offenkettigen Dihydroxycarbonsäuren der Formel II, deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

4. Pharmazeutisches Präparat, dadurch gekennzeichnet, daß es eine Verbindung gemäß Anspruch 1 enthält.

5. Verbindungen gemäß Anspruch 1 zur Prophylaxe und Therapie der Arteriosklerose und Hypercholesterinämie.

6. Phenole und Thiophenole der Formel III 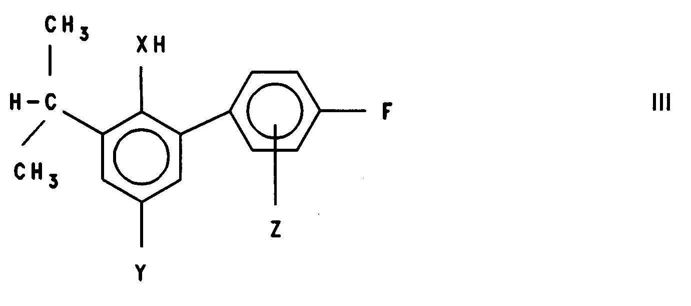 worin X, Y und Z die in Anspruch 1 zu den Formeln I und II angegebene Bedeutung haben.

## Patentansprüche (Patentansprüche für folgende(n) Vertragsstaat(en): ES)

1. Verfahren zur Herstellung von 6-Phenoxymethyl-4-hydroxytetrahydro-pyran-2-onen und 6-Thiophenoxymethyl-4-hydroxytetrahydropyran-2-onen der allgemeinen Formel I 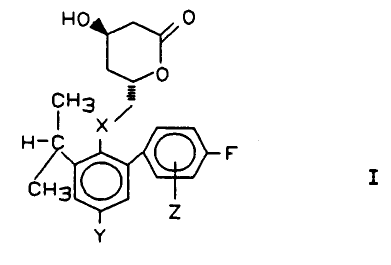 sowie von den entsprechenden offenkettigen Dihydroxycarbonsäuren der Formel II 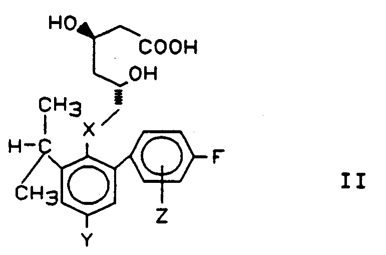 worin
X Sauerstoff oder Schwefel,
Y
a) einen geradkettigen oder verzweigten Alkylrest mit 3 bis 12 Kohlenstoffatomen
b) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 C-Atomen und
Z Wasserstoff oder einen geradkettigen oder verzweigten Alkylrest mit 1 bis 4 Kohlenstoffatomen,
bedeuten sowie von deren pharmakologisch verträglichen Salzen mit Basen und deren pharmakologisch verträglichen Estern dadurch gekennzeichnet, daß man
a) entsprechend substituierte Phenole oder Thiophenole der Formel III 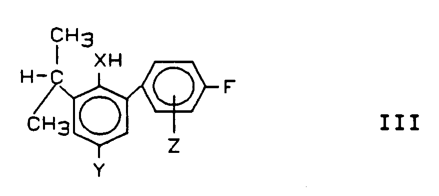 worin X, Y und Z die zu den Formeln I und II angegebenen Bedeutungen haben, mit dem optisch reinen Jodid der Formel IV 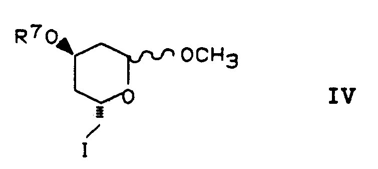 worin R⁷ eine gegen Basen und schwache Säuren stabile Schutzgruppe bedeutet, in die Lactolether der Formel V 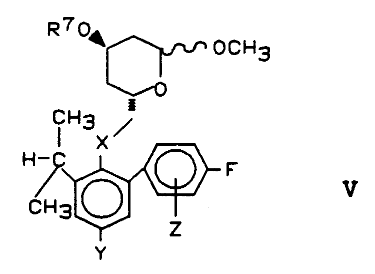 worin X, Y und Z die zu den Formeln I und II und R⁷ die zu Formel IV angegebenen Bedeutungen haben, überführt,
b) die Lactolether der Formel V zu den entsprechenden Lactolen der Formel VI 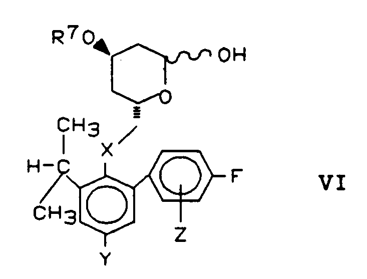 worin X, Y und Z die zu den Formeln I, II und R⁷ die zu Formel IV angegebene Bedeutungen haben, hydrolysiert,
c) die Lactole der Formel VI zu den entsprechenden Lactonen der Formel VII 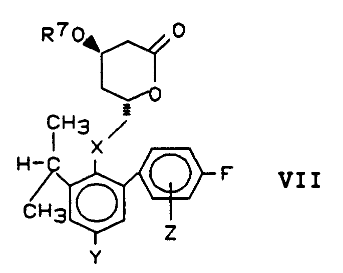 worin X, Y und Z die zu den Formeln I/II und R⁷ die zu Formel IV angegebenen Bedeutungen haben, oxydiert,
d) die erhaltenen geschützten Lactone der Formel VII in an sich bekannter Weise in Verbindungen der Formel I überführt und
e) gegebenenfalls die erhaltenen Verbindungen der Formel I in die entsprechenden offenkettigen Dihydroxycarbonsäuren der Formel II, deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

2. Verfahren gemäß Anspruch 1, dadurch gekennzeichnet, daß man Verbindungen der allgemeinen Formel I bzw. II, worin
X Sauerstoff
Y Isopropyl, tert.-Butyl, Cyclohexyl, Phenyl, p-Fluorphenyl
Z Wasserstoff bedeuten, oder deren pharmakologisch verträgliche Salze mit Basen oder deren pharmakologisch verträgliche Ester herstellt.

3. Verfahren zur Herstellung eines pharmazeutischen Präparates, dadurch gekennzeichnet, daß man eine Verbindung erhältlich gemäß Anspruch 1 in eine geeignete Darreichungsform überführt.

4. Verfahren gemäß Anspruch 1, dadurch gekennzeichnet, daß man Phenole oder Thiophenole der Formel III 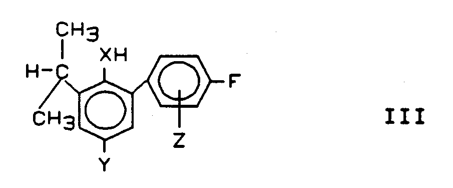 worin X, Y und Z die zu den Formeln I und II angegebene Bedeutung haben, isoliert.

## Patentansprüche (Patentansprüche für folgende(n) Vertragsstaat(en): GR)

1. Verfahren zur Herstellung von 6-Phenoxymethyl-4-hydroxytetrahydro-pyran-2-onen und 6-Thiophenoxymethyl-4-hydroxytetrahydropyran-2-onen der allgemeinen Formel I 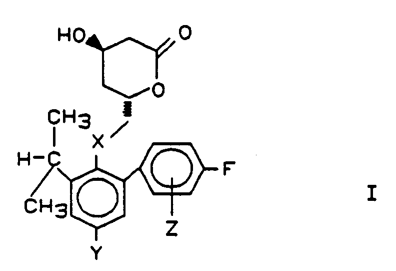 sowie von den entsprechenden offenkettigen Dihydroxycarbonsäuren der Formel II 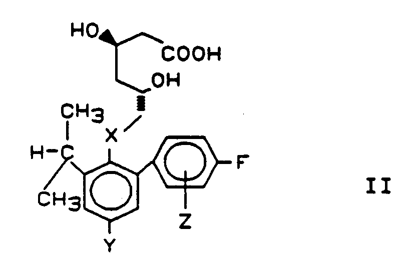 worin
X Sauerstoff oder Schwefel,
Y
a) einen geradkettigen oder verzweigten Alkylrest mit 3 bis 12 Kohlenstoffatomen
b) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 C-Atomen und
Z Wasserstoff oder einen geradkettigen oder verzweigten Alkylrest mit 1 bis 4 Kohlenstoffatomen,
bedeuten sowie von deren pharmakologisch verträglichen Salzen mit Basen und deren pharmakologisch verträglichen Estern dadurch gekennzeichnet, daß man
a) entsprechend substituierte Phenole oder Thiophenole der Formel III 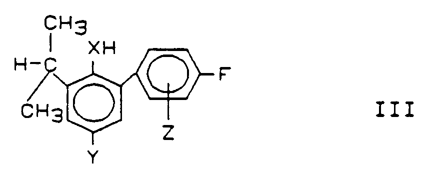 worin X, Y und Z die zu den Formeln I und II angegebenen Bedeutungen haben, mit dem optisch reinen Jodid der Formel IV 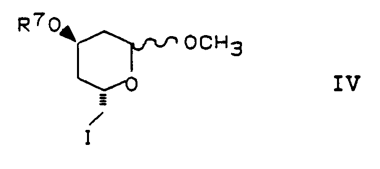 worin R⁷ eine gegen Basen und schwache Säuren stabile Schutzgruppe bedeutet, in die Lactolether der Formel V 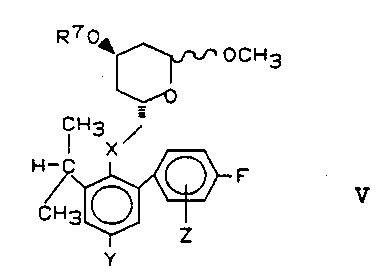 worin X, Y und Z die zu den Formeln I und II und R⁷ die zu Formel IV angegebenen Bedeutungen haben, überführt,
b) die Lactolether der Formel V zu den entsprechenden Lactolen der Formel VI 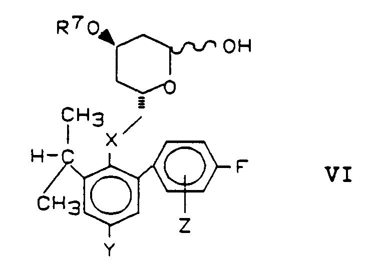 worin X, Y und Z die zu den Formeln I, II und R⁷ die zu Formel IV angegebene Bedeutungen haben, hydrolysiert,
c) die Lactole der Formel VI zu den entsprechenden Lactonen der Formel VII 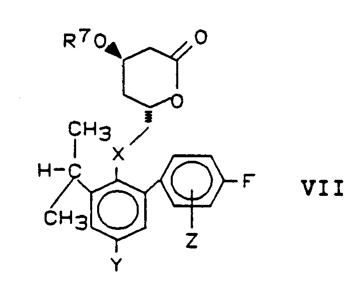 worin X, Y und Z die zu den Formeln I/II und R⁷ die zu Formel IV angegebenen Bedeutungen haben, oxydiert,
d) die erhaltenen geschützten Lactone der Formel VII in an sich bekannter Weise in Verbindungen der Formel I überführt und
e) gegebenenfalls die erhaltenen Verbindungen der Formel I in die entsprechenden offenkettigen Dihydroxycarbonsäuren der Formel II, deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

2. Verfahren gemäß Anspruch 1, dadurch gekennzeichnet, daß man Verbindungen der allgemeinen Formel I bzw. II, worin
X Sauerstoff
Y Isopropyl, tert.-Butyl, Cyclohexyl, Phenyl, p-Fluorphenyl
Z Wasserstoff bedeuten, oder deren pharmakologisch verträgliche Salze mit Basen oder deren pharmakologisch verträgliche Ester herstellt.

3. Verfahren zur Herstellung eines pharmazeutischen Präparates, dadurch gekennzeichnet, daß man eine Verbindung erhältlich gemäß Anspruch 1 in eine geeignete Darreichungsform überführt.

4. Phenole und Thiophenole der Formel III 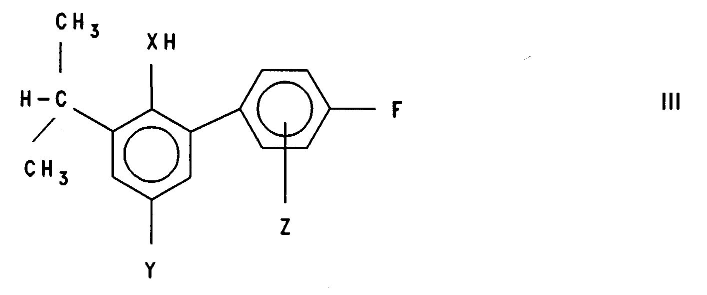 worin X, Y und Z die in Anspruch 1 zu den Formeln I und II angegebene Bedeutung haben.

## Claims (Claims for the following Contracting State(s): DE, GB, FR, IT, NL, SE, LI, CH, BE, AT, LU)

1. A 6-phenoxymethyl-4-hydroxy-tetrahydro-pyran-2-one or 6-thiophenoxymethyl-4-hydroxy-tetrahydropyran-2-one of the formula I 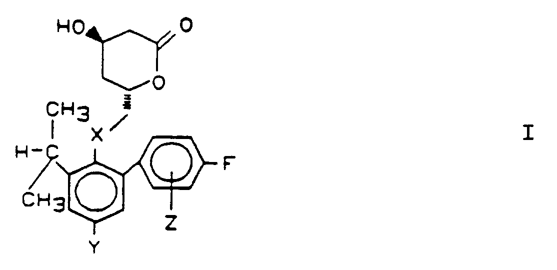 or a corresponding open-chain dihydroxycarboxylic acid of the formula II 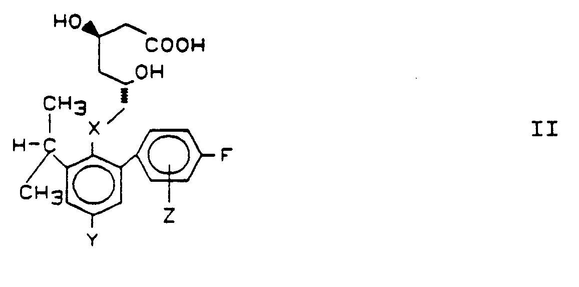 in which
X denotes oxygen or sulfur,
Y denotes
a) a straight-chain or branched alkyl radical having 3 to 12 carbon atoms or
b) cycloalkyl having 3 to 8 carbon atoms or a phenyl radical which can be substituted in the nucleus by 1 to 3 substituents from the group comprising halogen, trifluoromethyl and/or alkyl or alkoxy having in each case 1 to 4 carbon atoms and
Z denotes hydrogen or a straight-chain or branched alkyl radical having 1 to 4 carbon atoms,
or a pharmacologically tolerated salt thereof with a base or a pharmacologically tolerated ester thereof.

2. A compound as claimed in claim 1, wherein in the formula I or II
X denotes oxygen
Y denotes isopropyl, tert.-butyl, cyclohexyl, phenyl or p-fluorophenyl and
Z denotes hydrogen.

3. A process for the preparation of a compound of the formula I or a corresponding open-chain dihydroxycarboxylic acid of the formula II, a pharmacologically tolerated salt thereof with a base or a pharmacologically tolerated ester thereof as claimed in claim 1 which comprises
a) converting a correspondingly substituted phenol or thiophenol of the formula III 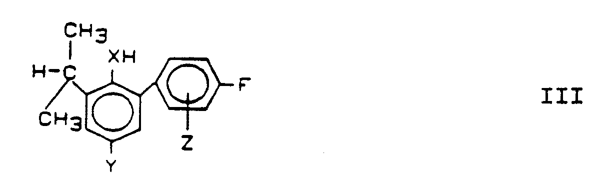 in which X, Y and Z have the meanings given in the case of formulae I and II, with the optically pure iodide of the formula IV 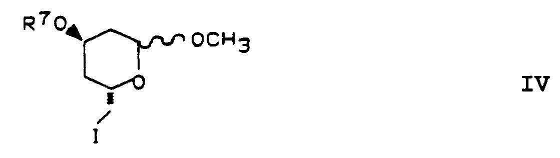 in which R⁷ denotes a protective group which is stable towards bases and weak acids, into the lactol ether of the formula V 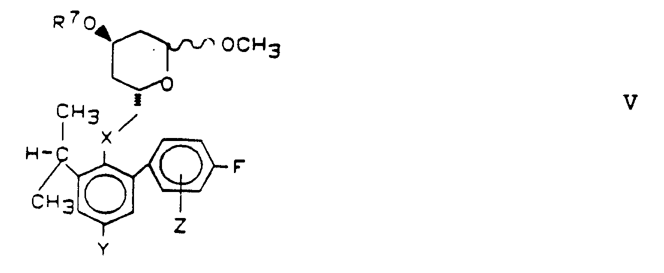 in which X, Y and Z have the meanings given in the case of formulae I and II and R⁷ has the meanings given in the case of formula IV,
b) hydrolyzing the lactol ether of the formula V to give the corresponding lactol of the formula VI 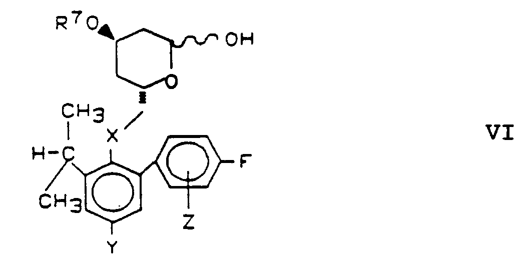 in which X, Y and Z have the meanings given in the case of formulae I and II and R⁷ has the meanings given in the case of formula IV,
c) oxidizing the lactol of the formula VI to give the corresponding lactone of the formula VII 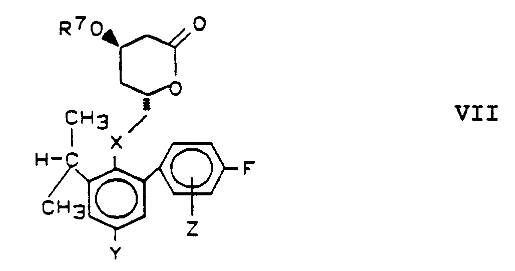 in which X, Y and Z have the meanings given in the case of formulae I and II and R⁷ has the meanings given in the case of formula IV,
d) converting the resulting protected lactone of the formula VII into a compound of the formula I in a manner which is known per se and,
e) if appropriate, converting the resulting compound of the formula I into the corresponding open-chain dihydroxycarboxylic acid of the formula II, or salt thereof or ester thereof, and if appropriate converting the resulting salt or ester into the free dihydroxycarboxylic acid or, if appropriate, converting the free carboxylic acid into a salt or ester.

4. A pharmaceutical preparation which contains a compound as claimed in claim 1.

5. A compound as claimed in claim 1 for the prophylaxis and therapy of arteriosclerosis and hypercholesterolemia.

6. A phenol or thiophenol of the formula III 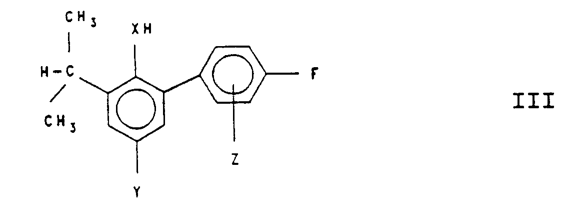 in which X, Y and Z have the meaning given in claim 1 in the case of formulae I and II.

## Claims (Claims for the following Contracting State(s): ES)

1. A process for the preparation of a 6-phenoxymethyl-4-hydroxy-tetrahydro-pyran-2-one or 6-thiophenoxymethyl-4-hydroxy-tetrahydropyran-2-one of the formula I 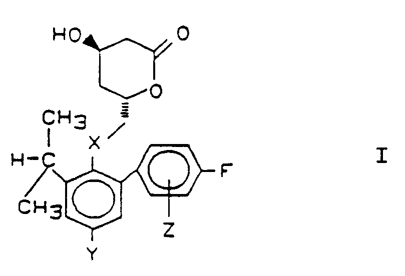 or a corresponding open-chain dihydroxycarboxylic acid of the formula II 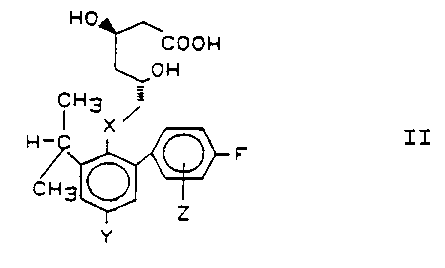 in which
X denotes oxygen or sulfur,
Y denotes
a) a straight-chain or branched alkyl radical having 3 to 12 carbon atoms or
b) cycloalkyl having 3 to 8 carbon atoms or a phenyl radical which can be substituted in the nucleus by 1 to 3 substituents from the group comprising halogen, trifluoromethyl and/or alkyl or alkoxy having in each case 1 to 4 carbon atoms and
Z denotes hydrogen or a straight-chain or branched alkyl radical having 1 to 4 carbon atoms,
or a pharmacologically tolerated salt thereof with a base or a pharmacologically tolerated ester thereof, which comprises
a) converting a correspondingly substituted phenol or thiophenol of the formula III 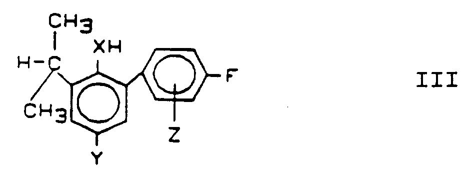 in which X, Y and Z have the meanings given in the case of formulae I and II, with the optically pure iodide of the formula IV 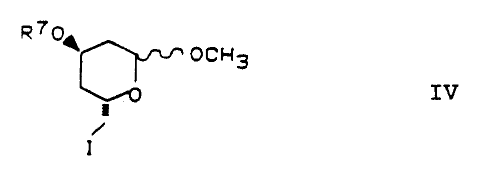 in which R⁷ denotes a protective group which is stable towards bases and weak acids, into the lactol ether of the formula V 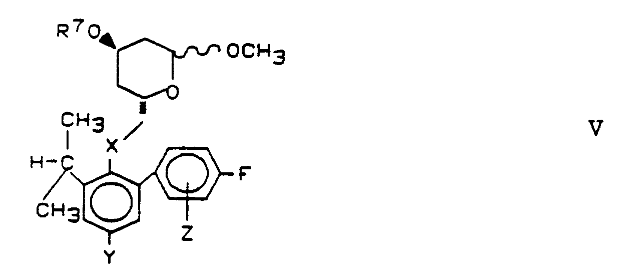 in which X, Y and Z have the meanings given in the case of formulae I and II and R⁷ has the meanings given in the case of formula IV,
b) hydrolyzing the lactol ether of the formula V to give the corresponding lactol of the formula VI 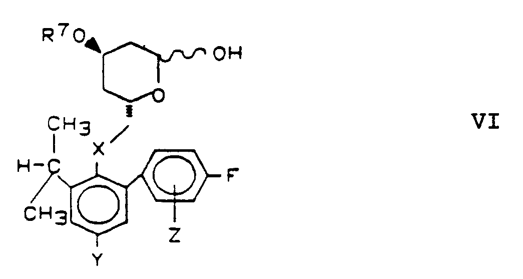 in which X, Y and Z have the meanings given in the case of formulae I and II and R⁷ has the meanings given in the case of formula IV,
c) oxidizing the lactol of the formula VI to give the corresponding lactone of the formula VII 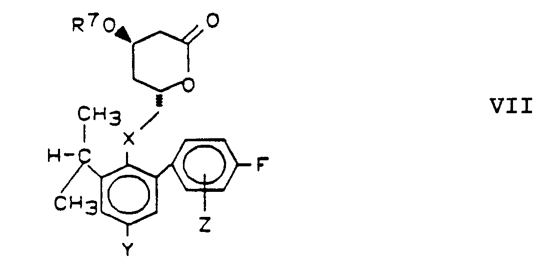 in which X, Y and Z have the meanings given in the case of formulae I and II and R⁷ has the meanings given in the case of formula IV,
d) converting the resulting protected lactone of the formula VII into a compound of the formula I in a manner which is known per se and,
e) if appropriate, converting the resulting compound of the formula I into the corresponding open-chain dihydroxycarboxylic acid of the formula II, or salt thereof or ester thereof, and if appropriate converting the resulting salt or ester into the free dihydroxycarboxylic acid or, if appropriate, converting the free carboxylic acid into a salt or ester.

2. The process as claimed in claim 1, which comprises preparing a commpound of the formula I or II in which
X denotes oxygen
Y denotes isopropyl, tert.-butyl, cyclohexyl, phenyl or p-fluorophenyl and
Z denotes hydrogen, or
a pharmacologically tolerated salt thereof with a base or a pharmacologically tolerated ester thereof.

3. A process for the preparation of a pharmaceutical preparation, which comprises converting a compound obtainable as claimed in claim 1 into a form suitable for administration.

4. The process as claimed in claim 1, which comprises isolating a phenol or thiophenol of the formula III 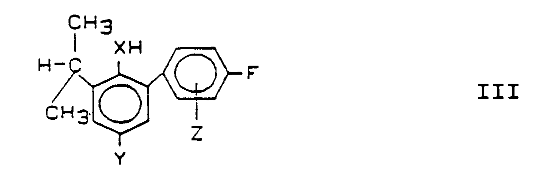 in which X, Y and Z have the meaning given in the case of formulae I and II.

## Claims (Claims for the following Contracting State(s): GR)

1. A process for the preparation of a 6-phenoxymethyl-4-hydroxy-tetrahydro-pyran-2-one or 6-thiophenoxymethyl-4-hydroxy-tetrahydropyran-2-one of the formula I 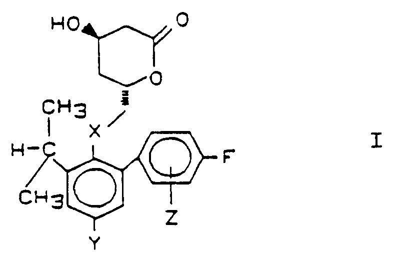 or a corresponding open-chain dihydroxycarboxylic acid of the formula II 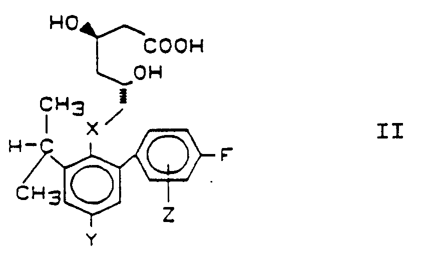 in which
X denotes oxygen or sulfur,
Y denotes
a) a straight-chain or branched alkyl radical having 3 to 12 carbon atoms or
b) cycloalkyl having 3 to 8 carbon atoms or a phenyl radical which can be substituted in the nucleus by 1 to 3 substituents from the group comprising halogen, trifluoromethyl and/or alkyl or alkoxy having in each case 1 to 4 carbon atoms and
Z denotes hydrogen or a straight-chain or branched alkyl radical having 1 to 4 carbon atoms,
or a pharmacologically tolerated salt thereof with a base or a pharmacologically tolerated ester thereof, which comprises
a) converting a correspondingly substituted phenol or thiophenol of the formula III 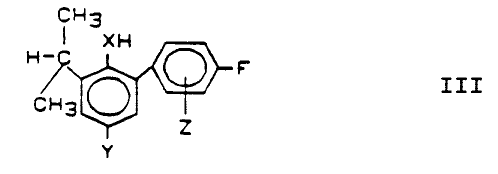 in which X, Y and Z have the meanings given in the case of formulae I and II, with the optically pure iodide of the formula IV 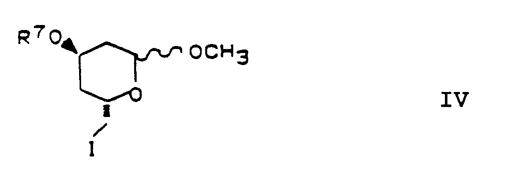 in which R⁷ denotes a protective group which is stable towards bases and weak acids, into the lactol ether of the formula V 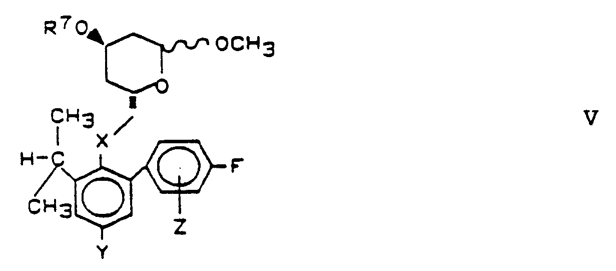 in which X, Y and Z have the meanings given in the case of formulae I and II and R⁷ has the meanings given in the case of formula IV,
b) hydrolyzing the lactol ether of the formula V to give the corresponding lactol of the formula VI 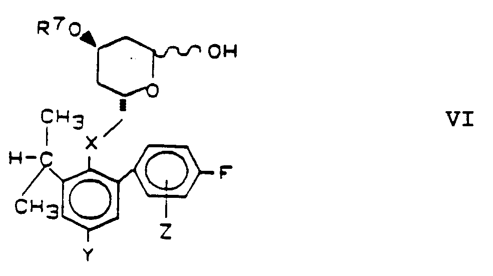 in which X, Y and Z have the meanings given in the case of formulae I and II and R⁷ has the meanings given in the case of formula IV,
c) oxidizing the lactol of the formula VI to give the corresponding lactone of the formula VII 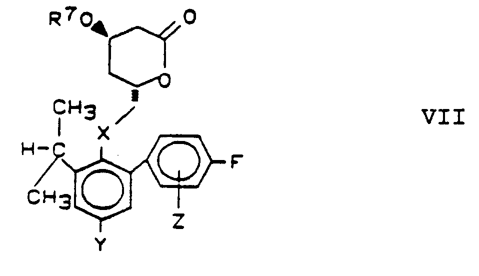 in which X, Y and Z have the meanings given in the case of formulae I and II and R⁷ has the meanings given in the case of formula IV,
d) converting the resulting protected lactone of the formula VII into a compound of the formula I in a manner which is known per se and,
e) if appropriate, converting the resulting compound of the formula I into the corresponding open-chain dihydroxycarboxylic acid of the formula II, or salt thereof or ester thereof, and if appropriate converting the resulting salt or ester into the free dihydroxycarboxylic acid or, if appropriate, converting the free carboxylic acid into a salt or ester.

2. The process as claimed in claim 1, which comprises preparing a compound of the formula I or II in which
X denotes oxygen
Y denotes isopropyl, tert.-butyl, cyclohexyl, phenyl or p-fluorophenyl and
Z denotes hydrogen, or
a pharmacologically tolerated salt thereof with a base or a pharmacologically tolerated ester thereof.

3. A process for the preparation of a pharmaceutical preparation, which comprises converting a compound obtainable as claimed in claim 1 into a form suitable for administration.

4. A phenol or thiophenol of the formula III 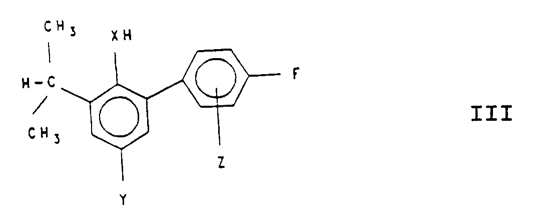 in which X, Y and Z have the meaning given in claim 1 in the case of formulae I and II.

## Revendications (Revendications pour l'(les) Etat(s) contractant(s) suivant(s): DE, GB, FR, IT, NL, SE, LI, CH, BE, AT, LU)

1. 6-phénoxyméthyl-4-hydroxy-tétrahydropyranne-2-ones et 6-thiophénoxyméthyl-4-hydroxy-tétrahydropyranne-2-ones de formule générale I 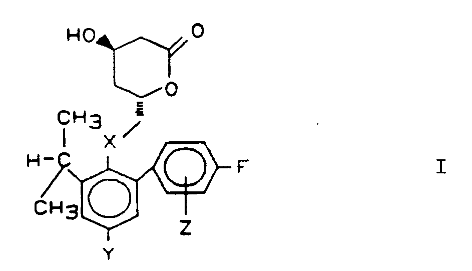 et acides dihydroxycarboxyliques à chaîne ouverte correspondants de formule II 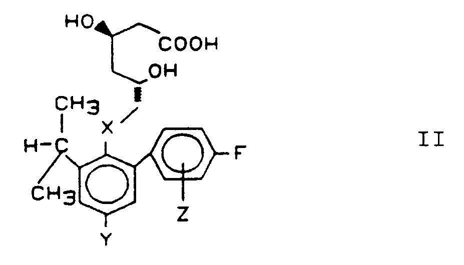 formules dans lesquelles
X représente un atome d'oxygène ou de soufre,
Y représente
a) un radical alkyle à chaîne droite ou ramifiée ayant de 3 à 12 atomes de carbone,
b) un radical cycloalkyle ayant de 3 à 8 atomes de carbone ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, et
Z représente un atome d'hydrogène ou un radical alkyle à chaîne droite ou ramifiée ayant de 1 à 4 atomes de carbone,
ainsi que leurs sels pharmacologiquement acceptables avec des bases et leurs esters pharmacologiquement acceptables.

2. Composés selon la revendication 1, caractérisés en ce que, dans les formules générales I ou II,
X représente un atome d'oxygène,
Y représente le radical isopropyle, tert-butyle, cyclohexyle, phényle, p-fluorophényle,
Z représente un atome d'hydrogène.

3. Procédé pour la préparation des composés de formule I ainsi que des acides dihydroxycarboxyliques à chaîne ouverte correspondants de formule II, de leurs sels pharmacologiquement acceptables avec des bases et de leurs esters pharmacologiquement acceptables selon la revendication 1, caractérisé en ce que
a) on convertit des phénols ou thiophénols convenablement substitués de formule III 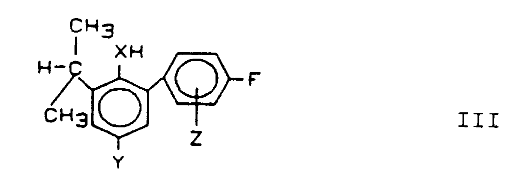 dans laquelle X, Y et Z ont les significations données dans les formules I et II, avec l'iodure optiquement pur de formule IV 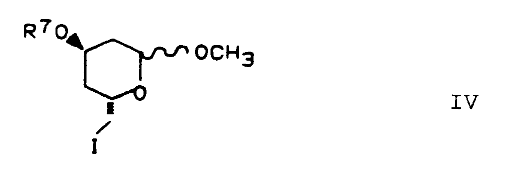 dans laquelle R⁷ représente un groupe protecteur stable en présence de bases et d'acide, faibles
en les éthers de lactol de formule V 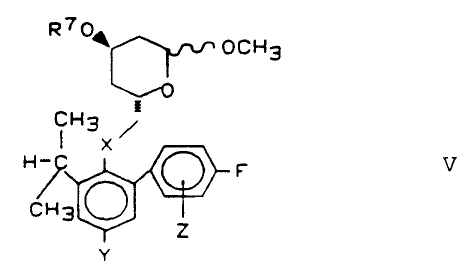 dans laquelle X, Y et Z ont les significations données à propos des formules I et II et R⁷ a les significations données à propos de la formule IV,
b) on hydrolyse les éthers de lactol de formule V en les lactols de formule VI correspondants 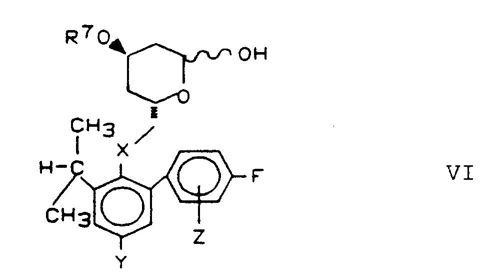 dans laquelle X, Y et Z ont les significations données à propos des formules I, II, et R⁷ a les significations données à propos de la formule IV,
c) on oxyde les lactols de formule VI en les lactones correspondantes de formule VII 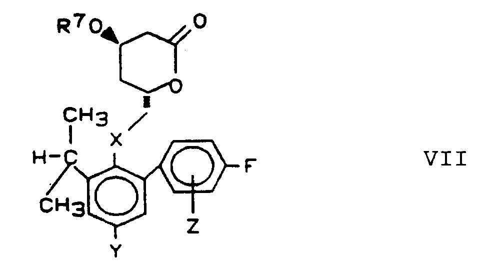 dans laquelle X, Y et Z ont les significations données à propos des formules I/II, et R⁷ a les significations données à propos de la formule IV,
d) on convertit d'une façon connue en soi les lactones protégées de formule VII obtenues en composés de formule I, et
e) éventuellement on convertit les composés de formule I obtenus en les acides dihydroxycarboxyliques à chaîne ouverte de formule II correspondants, leurs sels ou leurs esters, éventuellement on convertit des sels ou esters obtenus en les acides dihydroxycarboxyliques libres ou éventuellement on convertit les acides carboxyliques libres en les sels ou esters.

4. Composition pharmaceutique, caractérisée en ce qu'elle contient un composé selon la revendication 1.

5. Composés selon la revendication 1, pour la prophylaxie et le traitement de l'artériosclérose et de l'hypercholestérolémie.

6. Phénols et thiophénols de formule III 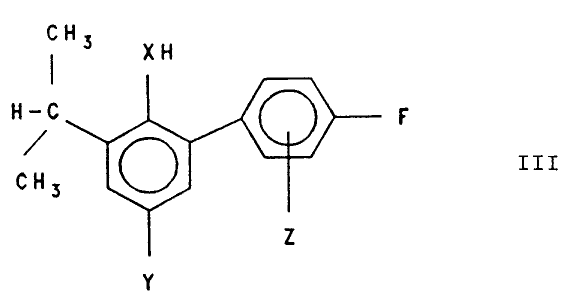 dans laquelle X, Y et Z ont les significations données à propos des formules I et II dans la revendication 1.

## Revendications (Revendications pour l'(les) Etat(s) contractant(s) suivant(s): ES)

1. Procédé pour la préparation de 6-phénoxyméthyl-4-hydroxy-tétrahydropyranne-2-ones et 6-thiophénoxyméthyl-4-hydroxy-tétrahydropyranne-2-ones de formule générale I 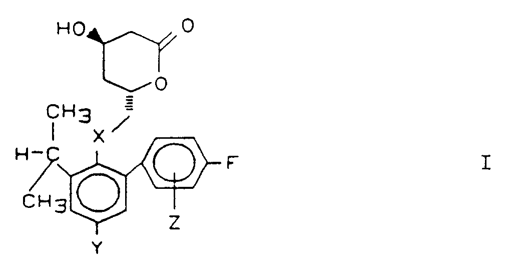 ainsi que des acides dihydroxycarboxyliques à chaîne ouverte correspondants de formule II 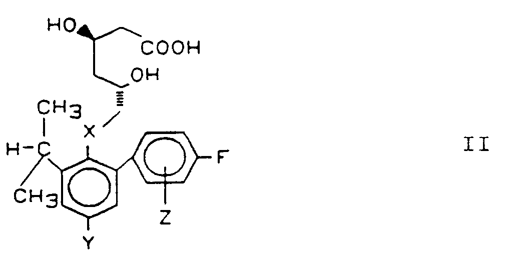 formules dans lesquelles
X représente un atome d'oxygène ou de soufre,
Y représente
a) un radical alkyle à chaîne droite ou ramifiée ayant de 3 à 12 atomes de carbone,
b) un radical cycloalkyle ayant de 3 à 8 atomes de carbone ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, et
Z représente un atome d'hydrogène ou un radical alkyle à chaîne droite ou ramifiée ayant de 1 à 4 atomes de carbone,
ainsi que de leurs sels pharmacologiquement acceptables avec des bases et de leurs esters pharmacologiquement acceptables, caractérisé en ce que
a) on convertit des phénols ou thiophénols convenablement substitués de formule III 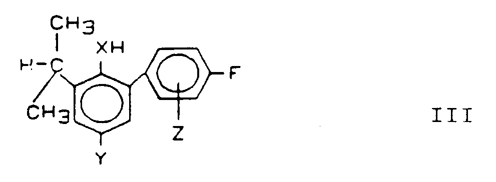 dans laquelle X, Y et Z ont les significations données dans les formules I et II, avec l'iodure optiquement pur de formule IV 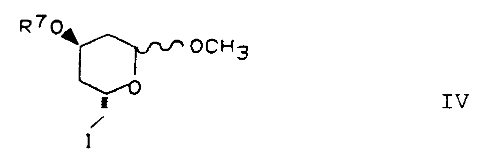 dans laquelle R⁷ représente un groupe protecteur stable en présence de bases et d'acides faibles en les éthers de lactol de formule V 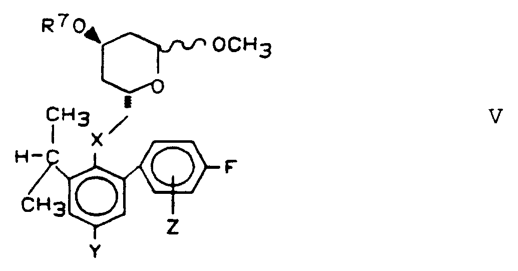 dans laquelle X, Y et Z ont les significations données à propos des formules I et II et R⁷ a les significations données à propos de la formule IV,
b) on hydrolyse les éthers de lactol de formule V en les lactols de formule VI correspondants 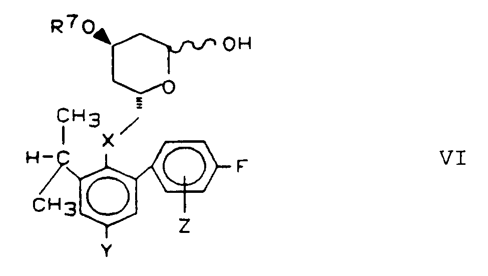 dans laquelle X, Y et Z ont les significations données à propos des formules I, II, et R⁷ a les significations données à propos de la formule IV,
c) on oxyde les lactols de formule VI en les lactones correspondantes de formule VII 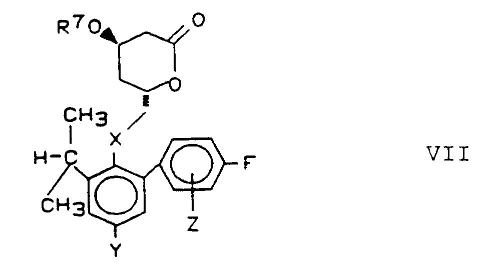 dans laquelle X, Y et Z ont les significations données à propos des formules I/II, et R⁷ a les significations données à propos de la formule IV,
d) on convertit d'une façon connue en soi les lactones protégées de formule VII obtenues en composés de formule I, et
e) éventuellement on convertit les composés de formule I obtenus en les acides dihydroxycarboxyliques à chaîne ouverte de formule II correspondants, leurs sels ou leurs esters, éventuellement on convertit des sels ou esters obtenus en les acides dihydroxycarboxyliques libres ou éventuellement on convertit les acides carboxyliques libres en les sels ou esters.

2. Procédé selon la revendication 1, caractérisé en ce que l'on prépare des composés de formule générale I ou II, dans lesquels
X représente un atome d'oxygène,
Y représente le radical isopropyle, tert-butyle, cyclohexyle, phényle, p-fluorophényle,
Z représente un atome d'hydrogène,
ou leurs sels pharmacologiquement acceptables avec des bases ou leurs esters pharmacologiquement acceptables.

3. Procédé pour la préparation d'une composition pharmaceutique, caractérisé en ce que l'on met sous une forme pharmaceutique appropriée un composé pouvant être obtenu selon la revendication 1.

4. Procédé selon la revendication 1, caractérisé en ce que l'on isole des phénols ou thiophénols de formule III 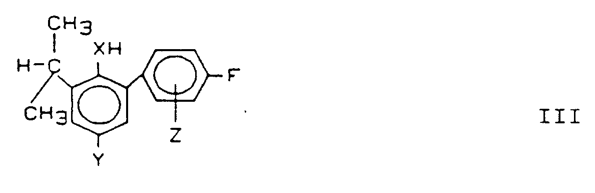 dans laquelle X, Y et Z ont les significations données à propos des formules I et II.

## Revendications (Revendications pour l'(les) Etat(s) contractant(s) suivant(s): GR)

1. Procédé pour la préparation de 6-phénoxyméthyl-4-hydroxy-tétrahydropyranne-2-ones et 6-thiophénoxyméthyl-4-hydroxy-tétrahydropyranne-2-ones de formule générale I 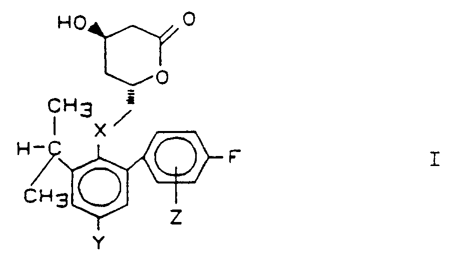 ainsi que des acides dihydroxycarboxyliques à chaîne ouverte correspondants de formule II 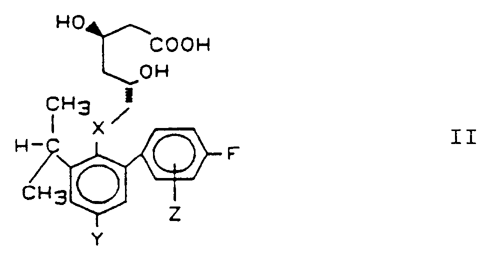 formules dans lesquelles
X représente un atome d'oxygène ou de soufre,
Y représente
a) un radical alkyle à chaîne droite ou ramifiée ayant de 3 à 12 atomes de carbone,
b) un radical cycloalkyle ayant de 3 à 8 atomes de carbone ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, et
Z représente un atome d'hydrogène ou un radical alkyle à chaîne droite ou ramifiée ayant de 1 à 4 atomes de carbone,
ainsi que de leurs sels pharmacologiquement acceptables avec des bases et de leurs esters pharmacologiquement acceptables, caractérisé en ce que
a) on convertit des phénols ou thiophénols convenablement substitués de formule III 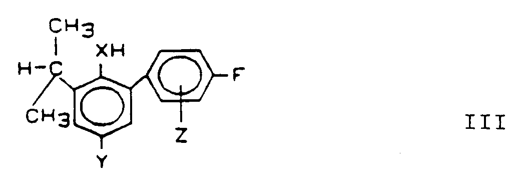 dans laquelle X, Y et Z ont les significations données dans les formules I et II, avec l'iodure optiquemont pur de formule IV 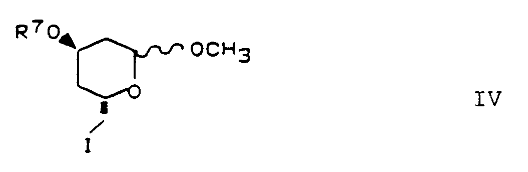 dans laquelle R⁷ représente un groupe protecteur stable en présence de bases et d'acides faibles en les éthers de lactol de formule V 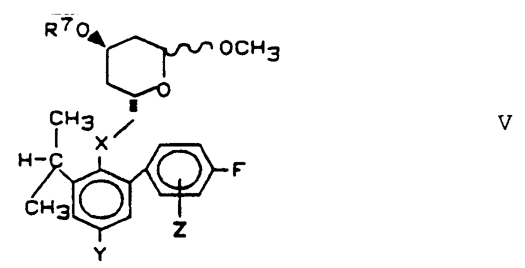 dans laquelle X, Y et Z ont les significations données à propos des formules I et II et R⁷ a les significations données à propos de la formule IV,
b) on hydrolyse les éthers de lactol de formule V en les lactols de formule VI correspondants 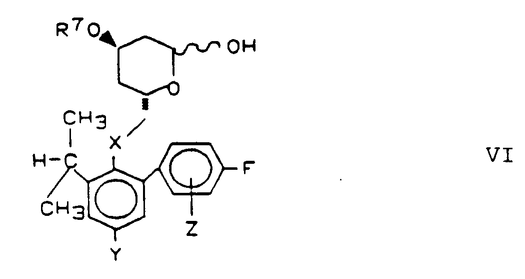 dans laquelle X, Y et Z ont les significations données à propos des formules I, II, et R⁷ a les significations données à propos de la formule IV,
c) on oxyde les lactols de formule VI en les lactones correspondantes de formule VII 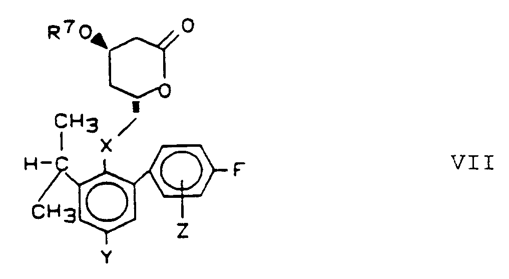 dans laquelle X, Y et Z ont les significations données à propos des formules I/II, et R⁷ a les significations données à propos de la formule IV,
d) on convertit d'une façon connue en soi les lactones protégées de formule VII obtenues en composés de formule I, et
e) éventuellement on convertit les composés de formule I obtenus en les acides dihydroxycarboxyliques à chaîne ouverte de formule II correspondants, leurs sels ou leurs esters, éventuellement on convertit des sels ou esters obtenus en les acides dihydroxycarboxyliques libres ou éventuellement on convertit les acides carboxyliques libres en les sels ou esters.

2. Procédé selon la revendication 1, caractérisé en ce que l'on prépare des composés de formule générale I ou II, dans lesquels
X représente un atome d'oxygène,
Y représente le radical isopropyle, tert-butyle, cyclohexyle, phényle, p-fluorophényle,
Z représente un atome d'hydrogène,
ou leurs sels pharmacologiquement acceptables avec des bases ou leurs esters pharmacologiquement acceptables.

3. Procédé pour la préparation d'une composition pharmaceutique, caractérisé en ce que l'on met sous une forme pharmaceutique appropriée un composé pouvant être obtenu selon la revendication 1.

4. Phénols et thiophénols de formule III 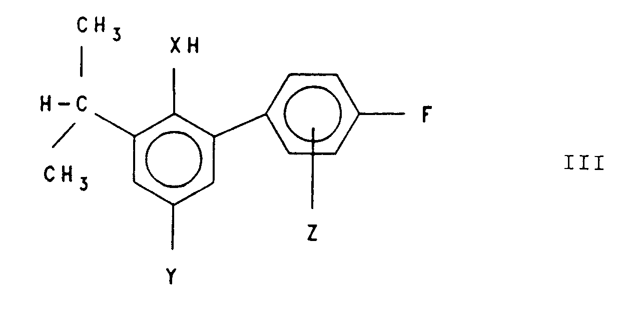 dans laquelle X, Y et Z ont les significations données à propos des formules I et II dans la revendication 1.
